# EUROPEAN PATENT APPLICATION

(11) **EP 0 970 968 A1**
(43) Date of publication of application: **12.01.2000**
(21) Application number: 98911077.0
(22) Date of filing: 27.03.1998
(51) Int. Cl.: C08B 37/00, A61K 35/78

(54) **PHARMACOLOGICALLY ACTIVE SUBSTANCE**

(30) Priority: 28.03.1997 JP 7860397; 17.09.1997 JP 25254897
(71) Applicant: The Kampou Foundation, Tokyo 160-0022 (JP)
(72) Inventor: NAGAI, Kazuo, Tokyo 171-0014 (JP); KATAOKA, Takao, Tokyo 169-0074 (JP); OTAKE, Noboru, Kanagawa 240-0025 (JP); NISHIMURA, Toshio, Tochigi 320-0003 (JP); ABE, Shigeru, Tokyo 173-0004 (JP)
(74) Representative: Jorio, Paolo, Dr. Ing.
(86) International application number: JP9801416
(87) International publication number: WO9844005

(57) **Abstract**

From a hot water extract of a safflower, substances having a polysaccaride comprising rhamnose, arabinose, xylose, mannose, glucose and galactose are isolated. Such substances show immunoactivity, IL-1 production promoting activity, IL-6 production promoting activity, TNF-α production promoting, carcinostatic action, cancer metastasis-inhibiting action, and phylaxis action, and can be expected to be used as a medical product. The above-mentioned composition of the polysaccharide contained in such pharmacologically active substances comprise 2.9 of rhamnose in terms of a weight ratio to 1.0 of mannose. Thus, other than the medical products, it can be considered to use them as an additive for foods.

## Description

### Technical field

This invention relates to novel pharmacologically active substances comprising macromolecular polysaccharides obtained from an extract of a safflower as effective ingredients and showing various medicinal effects and pharmacological effects such as immunoactivity, carcinostatic action, cancer metastasis-inhibiting action, phylaxis property, etc.

### Background art

It has heretofore been empirically known that a safflower has various effects of a medicine in the field of Chinese medicine. The present inventor has found that such a safflower has carcinostatic action. However, effective ingredients in the safflower showing such a carcinostatic effect are natural macromolecular substance so that the molecular structures thereof have not yet been determined.

However, in a viewpoint of attempting relief of tumor patients such as cancer, etc. even it is a little, it would be a pressing need for contributing to treatment by effecting isolation and purification of a substance(s) having such a carcinostatic effect even when the molecular structure has not yet determined. Incidentally, in Japanese Patent Laid-open No. 60-11892, and Japanese Patent Laid-open No. 61-52807, there are description about a composition for inducing interferon derived from a safflower extract.

Also, in the field of Chinese medicine, it has heretofore been known that there are various effects of a medicine in a safflower such as vasodilating effects or nourishment and vigorousness effects, etc., and as described above, the inventor considered that it would be necessary to carry out isolation and purification of a substance(s) having carcinostatic action from a safflower as well as widely inspecting presence or absence of a pharmacological action of the substance(s) other than the carcinostatic action to determine whether it could be applied to treatment other than antitumor or not. Moreover, it is necessary to carry out formation of a preparation so as to apply to actual treatment. Also, in the viewpoint not from a medical agent but from ISHOKU-DOGEN (the meaning that curing a disease and having a meal are substantially the same in view of keeping good health), or YAKUZEN (a meal combined with a herbal medicine and normal food materials for improving health or preventing and curing a disease), the inventor considered that it would be also necessary to search for a way to apply to a health supplementary foods to obtain an effect through usual dietary life.

Accordingly, an object of the present invention is to provide a novel pharmacologically active substance comprising a macromolecular polysaccharide of a safflower extract as an effective ingredient.

An object of the present invention is to provide a novel immunoactive agent comprising a macromolecular polysaccharide of a safflower extract as an effective ingredient.

An object of the present invention is to provide a novel carcinostatic agent comprising a macromolecular polysaccharide of a safflower extract as an effective ingredient.

An object of the present invention is to provide a novel cancer metastasis-inhibiting agent comprising a macromolecular polysaccharide of a safflower extract as an effective ingredient.

An object of the present invention is to provide a novel phylaxis agent comprising a macromolecular polysaccharide of a safflower extract as an effective ingredient.

An object of the present invention is to provide a novel IL-1 productive promoter comprising a macromolecular polysaccharide of a safflower extract as an effective ingredient.

An object of the present invention is to provide a novel IL-6 productive promoter comprising a macromolecular polysaccharide of a safflower extract as an effective ingredient.

An object of the present invention is to provide a novel TNF-α productive promoter comprising a macromolecular polysaccharide of a safflower extract as an effective ingredient.

An object of the present invention is to provide a food to which a novel pharmacological active substance comprising a macromolecular polysaccharide of a safflower extract as an effective ingredient is added.

### Disclosure of the invention

The pharmacologically active substances of the present invention show immunoactivities such as an IL-1 production promoting activity, TNF-α production promoting, IL-6 production promoting activity, etc., carcinostatic action, cancer metastasis-inhibiting action, phylaxis action, etc. and obtained as a hot water extract of a safflower. The pharmacologically active substances of the present invention contain a polysaccharide in which a plural number of saccharides (monosaccharides) are bound to each other, particularly a macromolecular polysaccharide as an effective ingredient, and the macromolecular polysaccharide contains rhamnose, arabinose, xylose, mannose, glucose and galactose as the saccharides.

A substance having such a saccharide composition is a novel substance different from the substances disclosed in Japanese Patent Laid-open No. 60-11892 or Japanese Patent Laid-open No. 61-52807 as a composition for inducing interferon derived from a safflower extract since the saccharide compositions are different from each other. Particularly, the macromolecular polysaccharide according to the present invention contains rhamnose in the saccharide composition whereas rhamnose is not contained in the above-mentioned composition for inducing interferon.

A petal of a safflower is extracted with hot water, ethanol is added to supernatant of the hot water extract to precipitate and the precipitate is freeze-dried and then subjected to the gel filtration method to obtain two fractions. In these fractions, the monosaccharides with the above-mentioned compositions are contained as effective ingredients, respectively, and the respective compositions of the monosaccharides are determined by gas chromatography, etc.

One of the above-mentioned fractions contains, in terms of a weight ratio, 2.9 of rhamnose, 7.5 of arabinose, 3.8 of xylose, 1.0 of mannose, 11.6 of glucose and 8.9 of galactose, and is a macromolecular polysaccharide in which these monosaccharides are bound to each other (the fraction having the saccharide composition is hereinafter called to SF1).

And the saccharide composition of the other fraction is, in terms of a weight ratio, 2.9 of rhamnose, 10.3 of arabinose, 4.2 of xylose, 1.0 of mannose, 5.1 of glucose and 8.5 of galactose, and is a macromolecular polysaccharide in which these monosaccharides are bound to each other (the fraction having the saccharide composition is hereinafter called to SF2). Incidentally, in the above-mentioned SF1 and SF2, rhamnose is contained in a weight ratio of 2.9 to 1.0 of mannose, respectively.

Immunoactivities of such fractions SF1 and SF2 were inspected by examining blastogenic activity and production of IgM after adding the above-mentioned SF1 and SF2 to cells extracted form spleen of C57BL/6 mouse.

Also, to have immunoactivity was inspected by injecting Thioglycolate to C3H/HeN mouse and determining production promoting activities of IL-1, TNF-α, NO and IL-6 of SF1 and SF2 against PEC of the mouse after lapsing predetermined days.

Moreover, carcinostatic action and cancer metastasis-inhibiting action were inspected by measuring survival days of mice in the administered groups of SF1 and SF2 before and after implantation and a number of colonies of B16 cells metastasized to lung through implantation of FM3A cells of ascites type mouse breast carcinoma to C3H/HeN mouse and implantation of melanoma B16 cells to BDF1 mouse.

A conventionally known substance showing carcinostatic action is a substance directly acting on cancer cells and many of them show side effects which are harmful also to normal cells. On the other hand, detailed mechanisms of the cancer metastasis-inhibiting action have not yet been elucidated, and it is different from the carcinostatic action. Even when it has the efficacy of a medicine as a carcinostatic agent, the efficacy of a medicine as the cancer metastasis-inhibiting property cannot be admitted with significant difference in many cases. The substance of the present invention shows a remarkable cancer metastasis-inhibiting action in addition to the carcinostatic action.

Moreover, in addition to the above-mentioned carcinostatic action and cancer metastasis-inhibiting action, a phylaxis action was also inspected.

As the effects of the present invention, the following effects can be inspected.
(1). A substance containing a macromolecular polysaccharide comprising rhamnose, arabinose, xylose, mannose, glucose and galactose shows a pharmacological activity.
(2). A substance containing a macromolecular polysaccharide comprising rhamnose, arabinose, xylose, mannose, glucose and galactose shows an effective immunoactivity.
(3). A substance containing a macromolecular polysaccharide comprising rhamnose, arabinose, xylose, mannose, glucose and galactose shows an effective carcinostatic action.
(4). A substance containing a macromolecular polysaccharide comprising rhamnose, arabinose, xylose, mannose, glucose and galactose shows an effective cancer metastasis-inhibiting action.
(5). A substance containing a macromolecular polysaccharide comprising rhamnose, arabinose, xylose, mannose, glucose and galactose shows an effective IL-1 production promoting activity.
(6). A substance containing a macromolecular polysaccharide comprising rhamnose, arabinose, xylose, mannose, glucose and galactose shows an effective IL-6 production promoting activity.
(7). A substance containing a macromolecular polysaccharide comprising rhamnose, arabinose, xylose, mannose, glucose and galactose shows an effective TNF-α production promoting activity.
(8). A substance containing a macromolecular polysaccharide comprising rhamnose, arabinose, xylose, mannose, glucose and galactose shows an effective phylaxis action.
(9). By adding a substance containing a macromolecular polysaccharide comprising rhamnose, arabinose, xylose, mannose, glucose and galactose to foods, the above-mentioned various effects can be expected through the foods.

### Brief explanation of the drawings

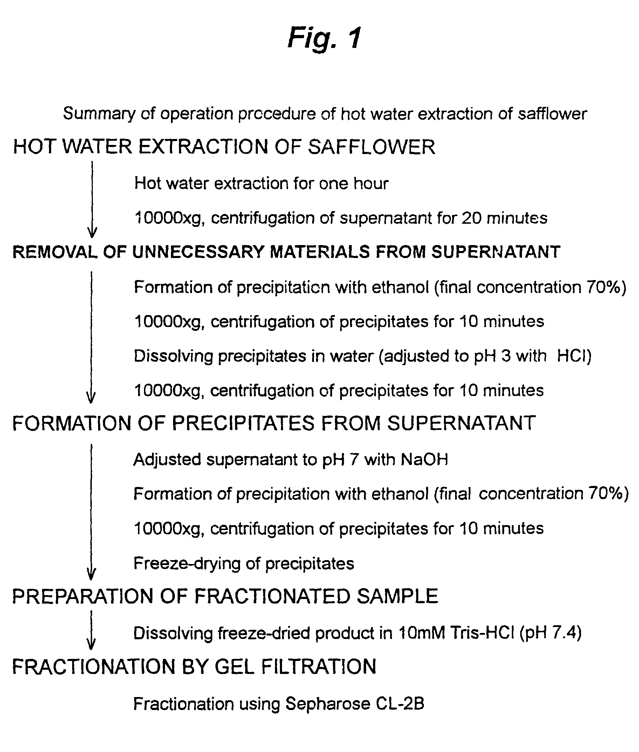

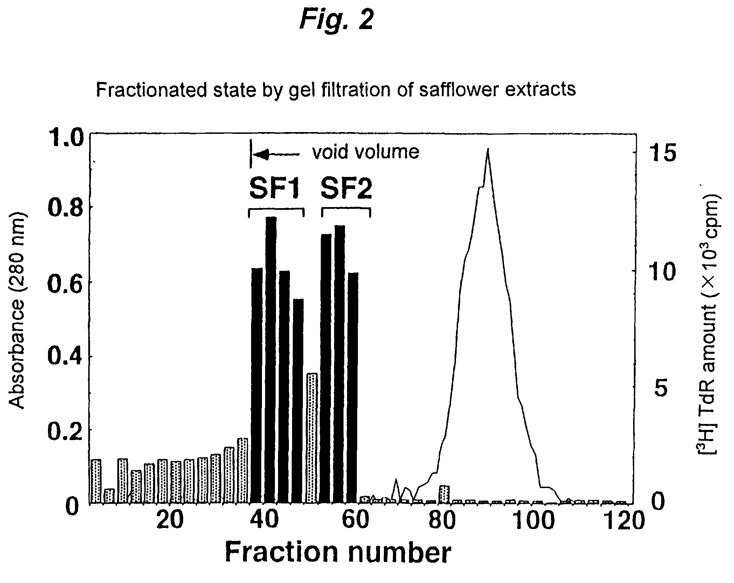

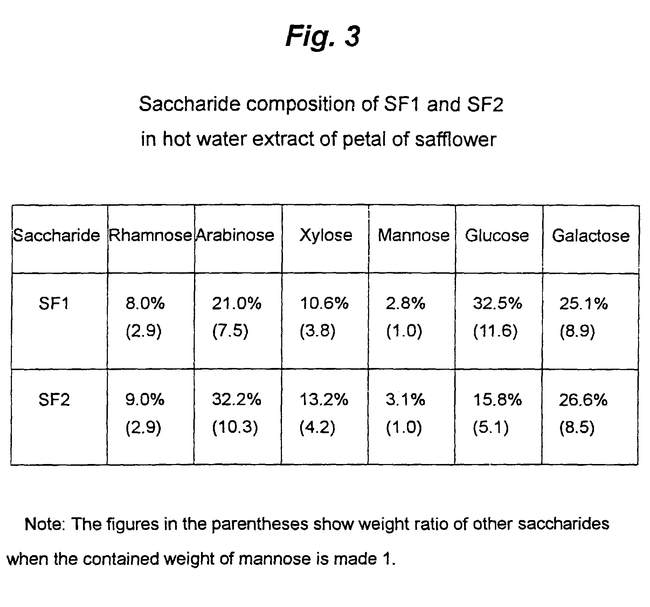

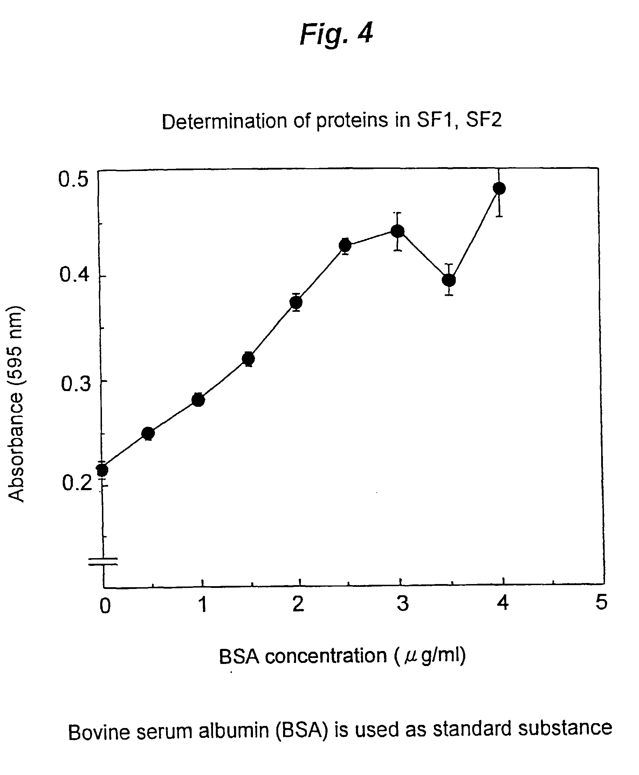

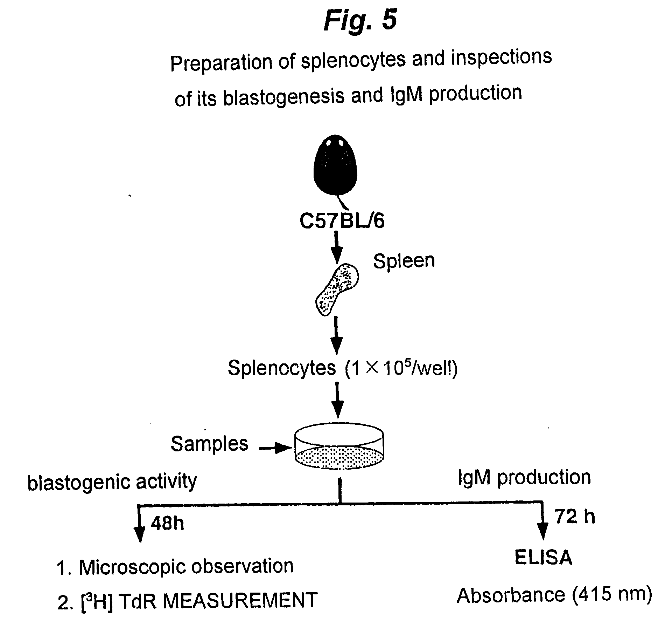

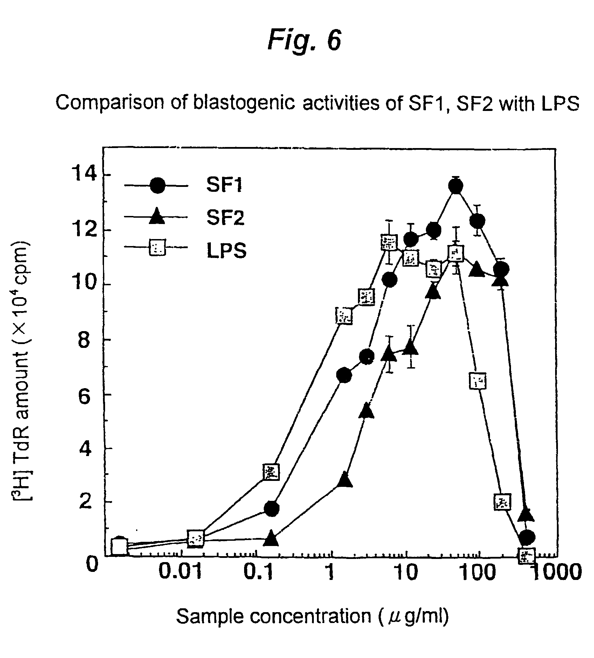

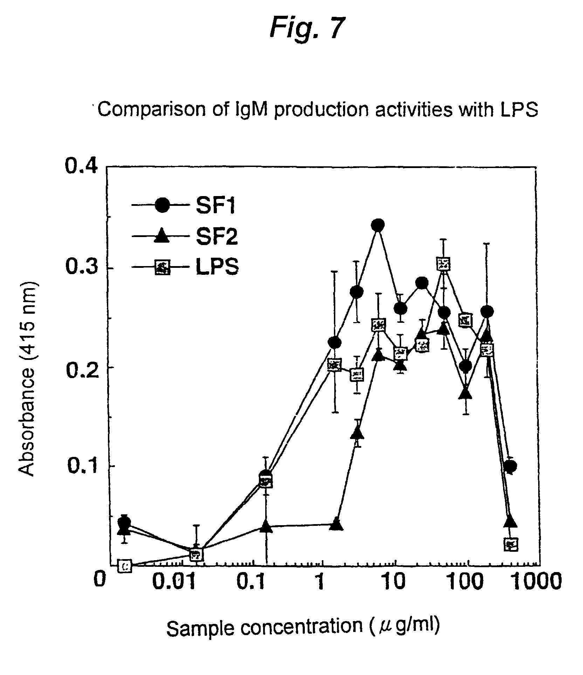

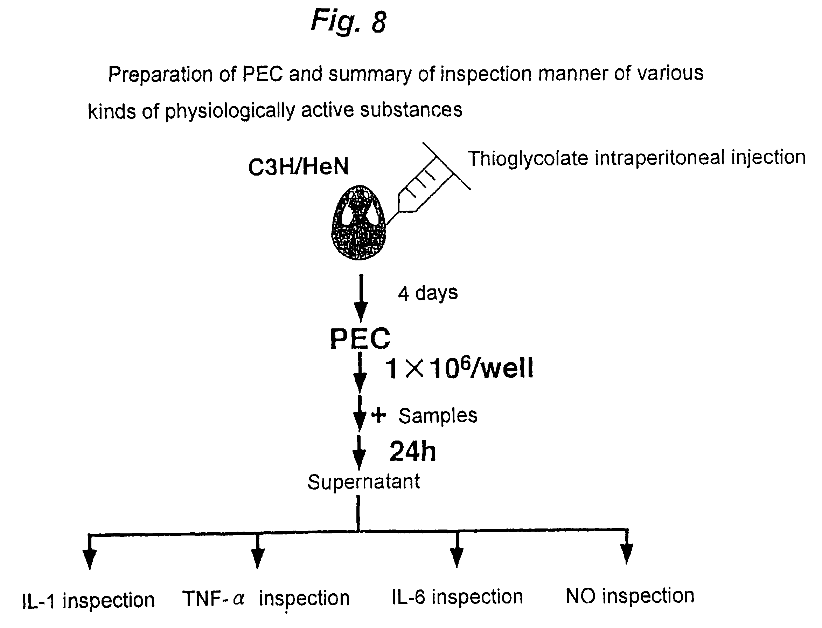

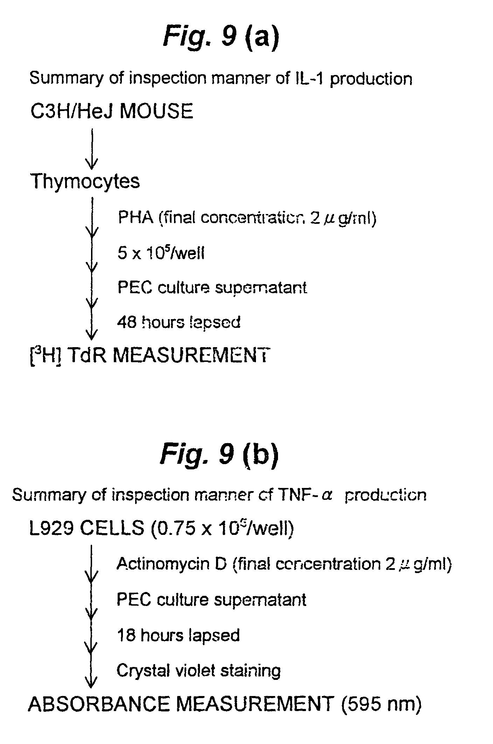

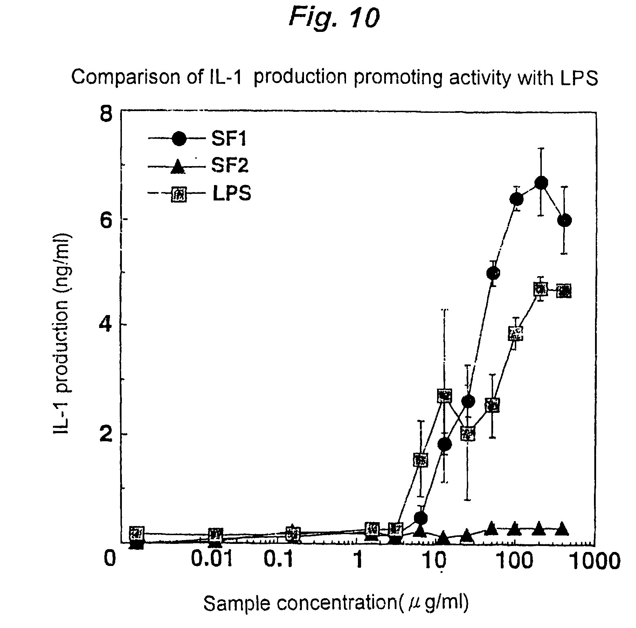

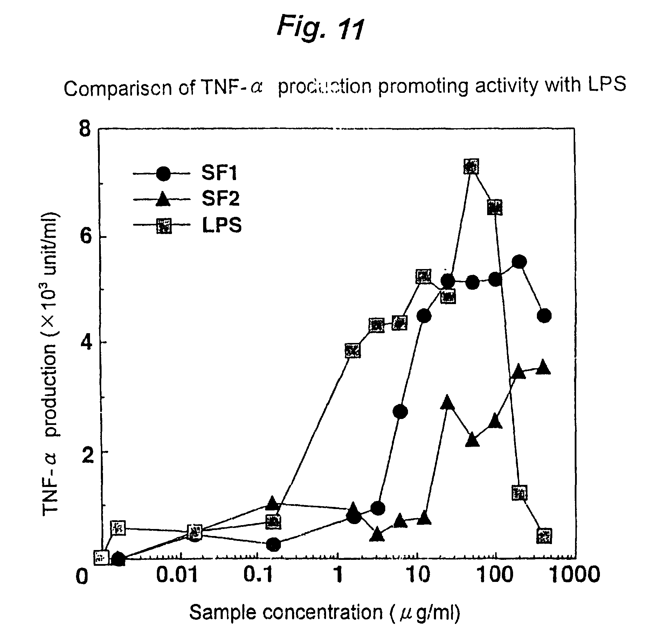

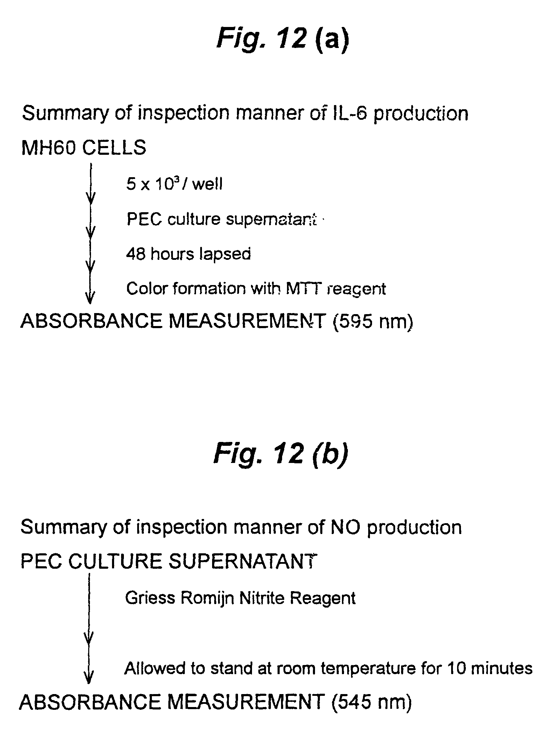

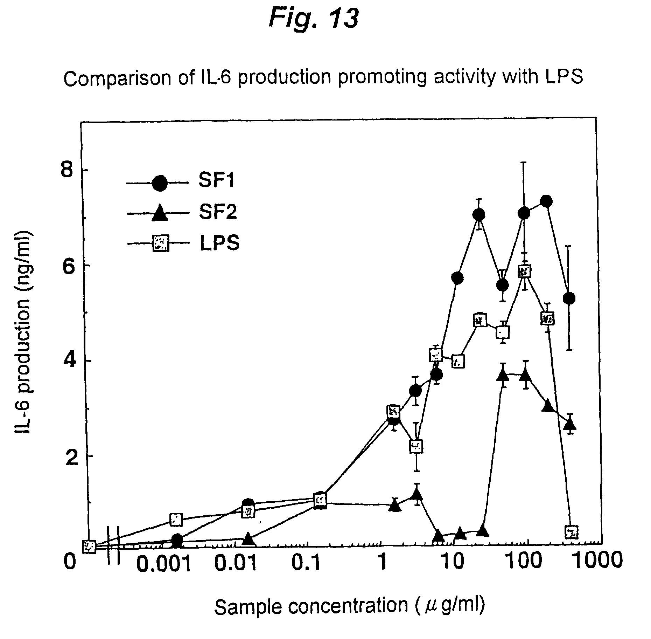

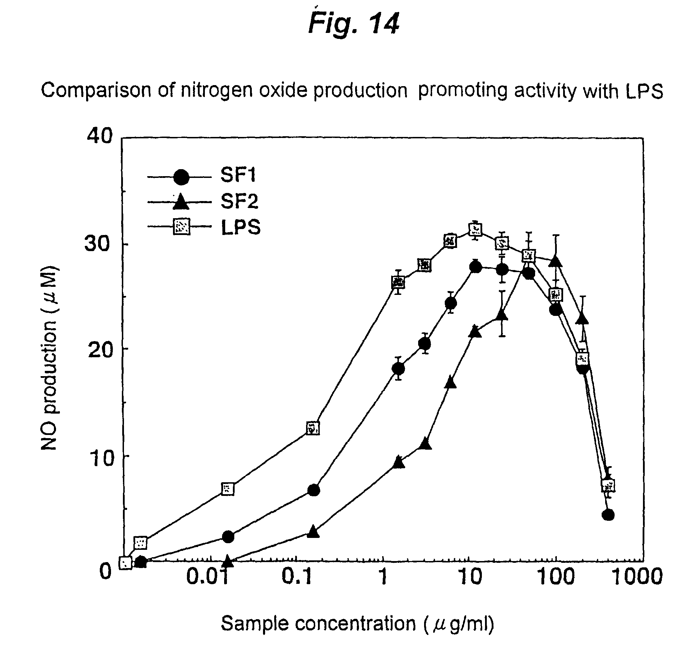

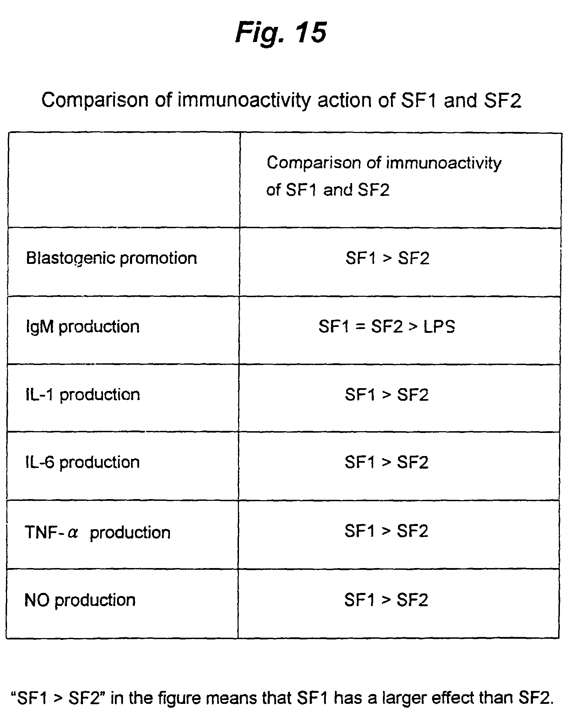

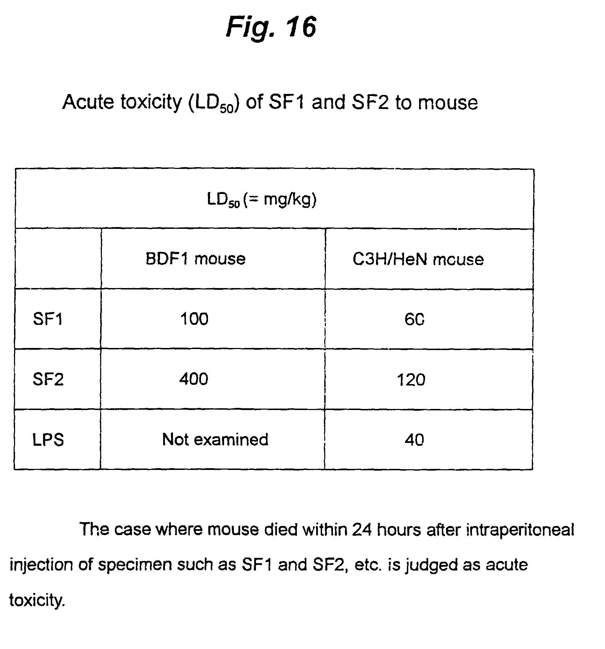

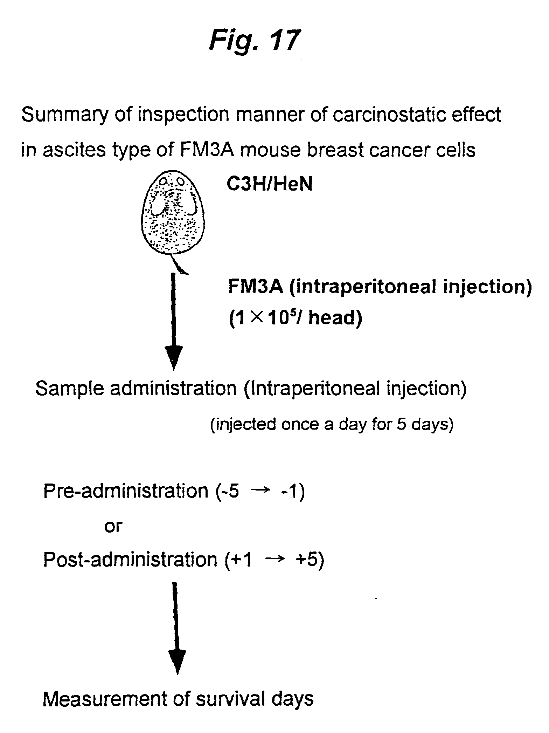

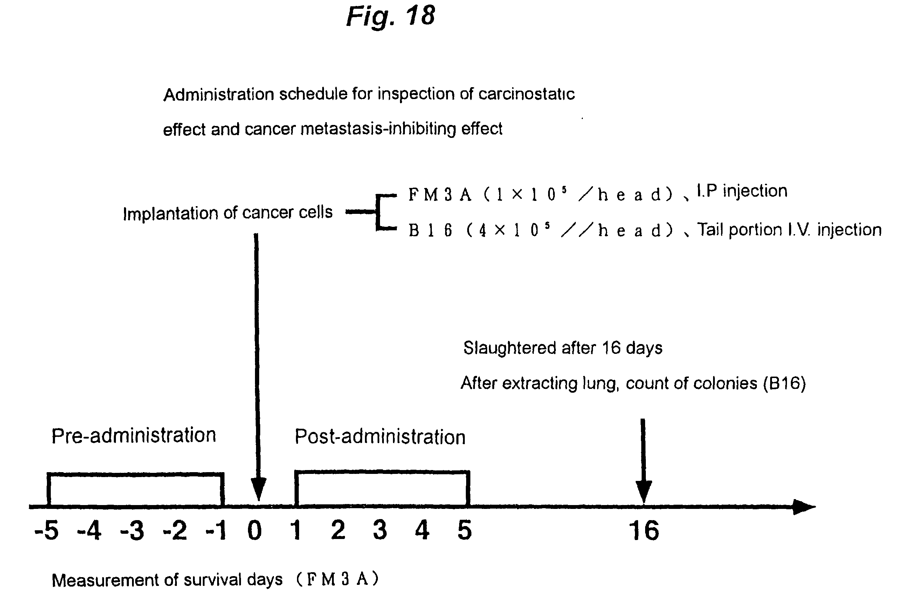

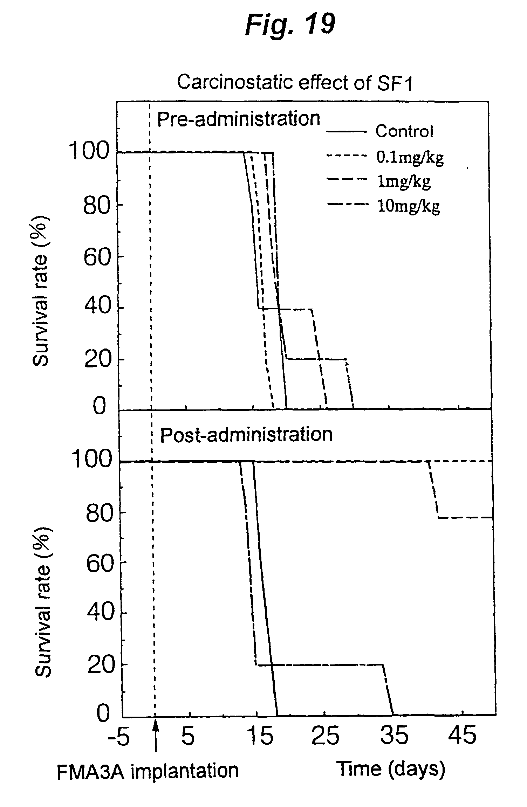

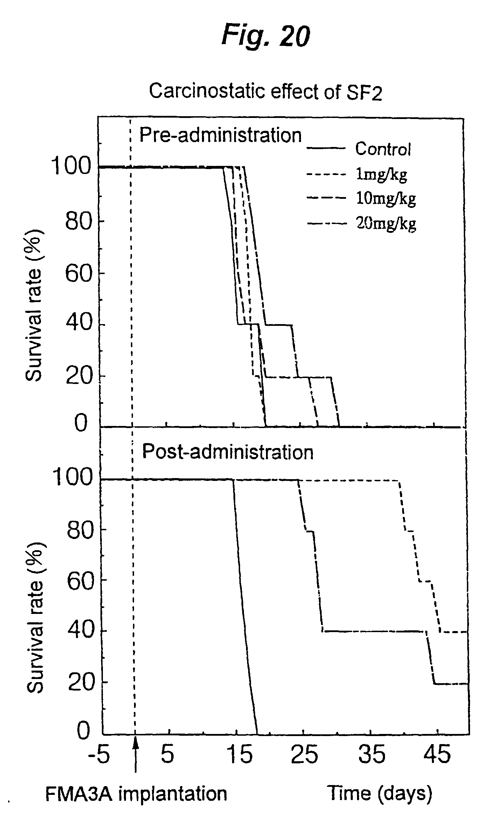

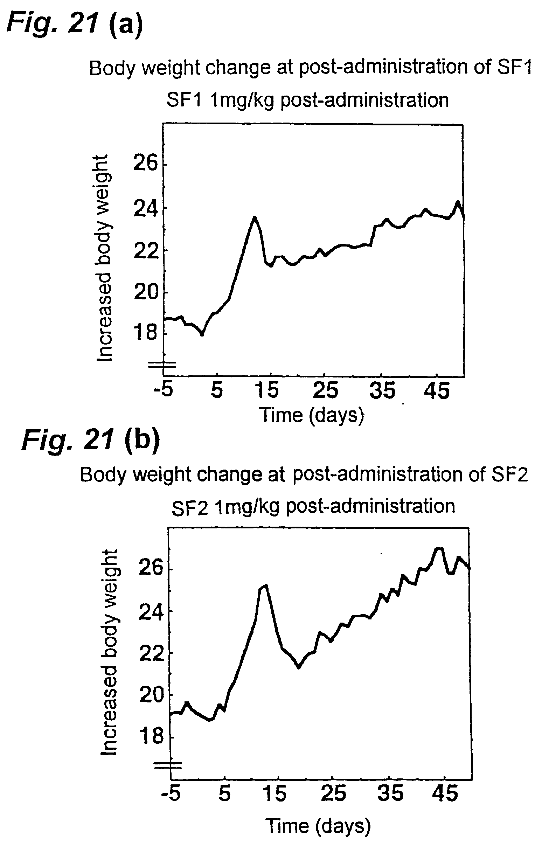

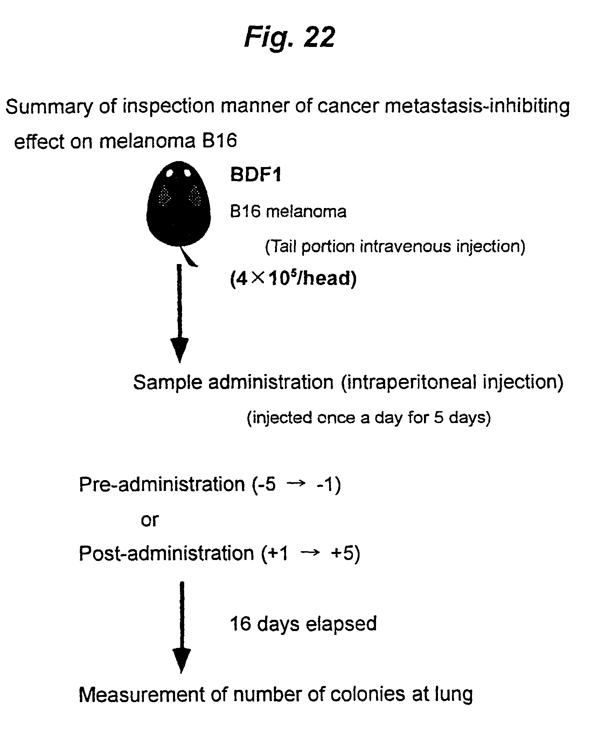

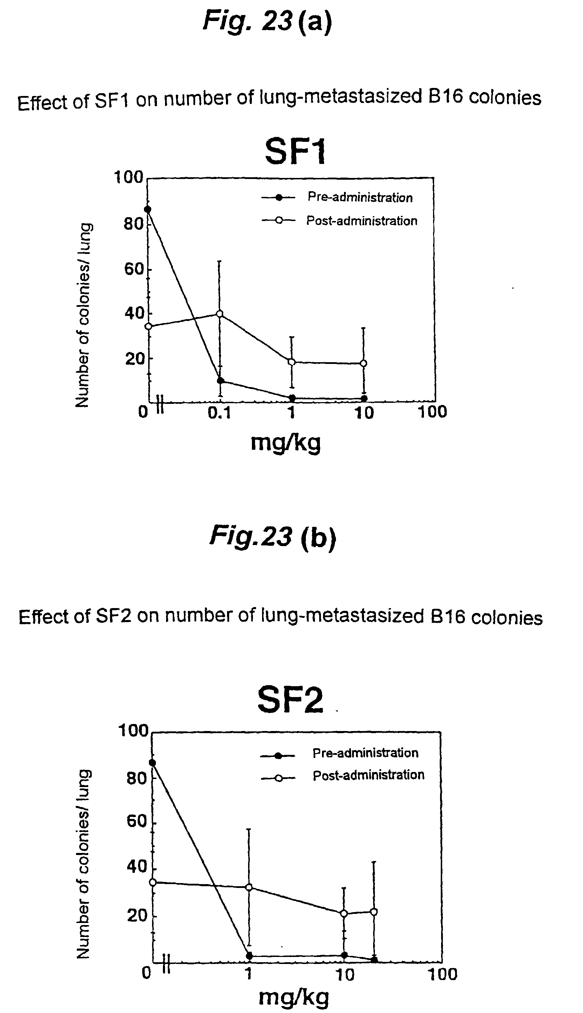

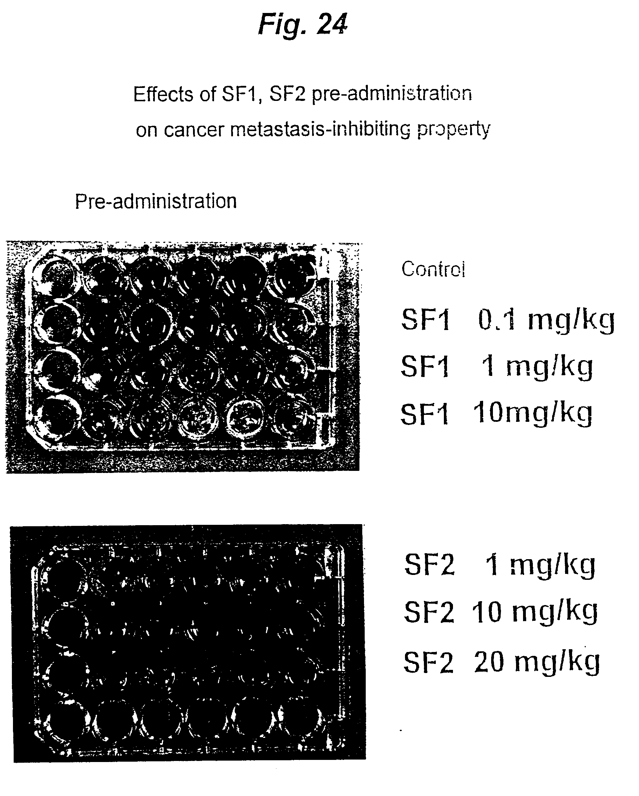

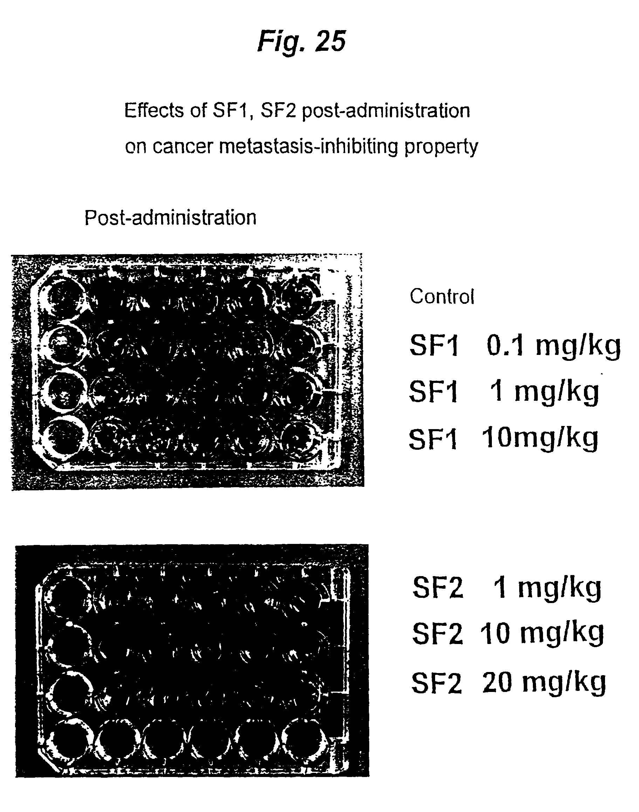

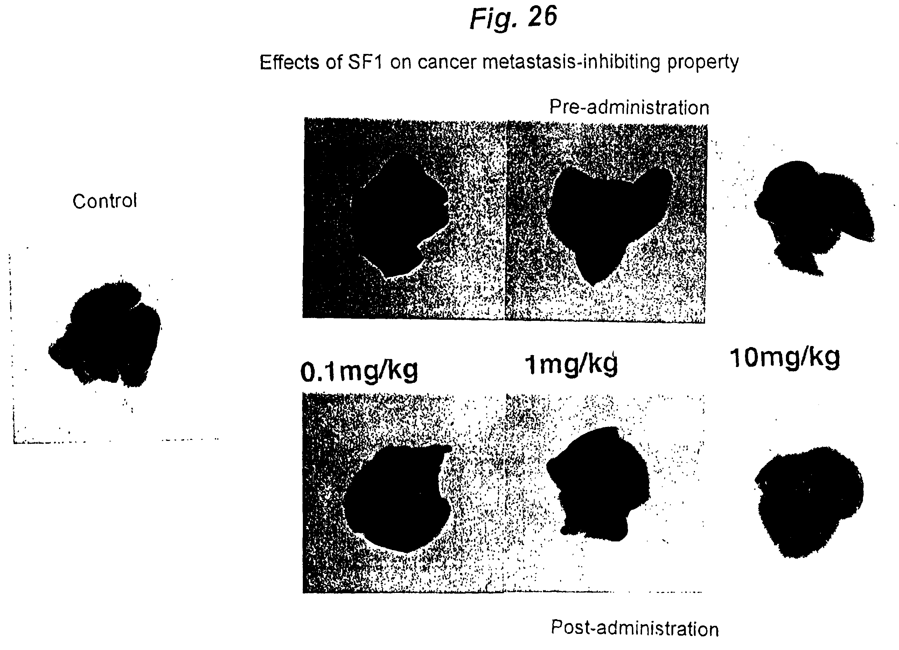

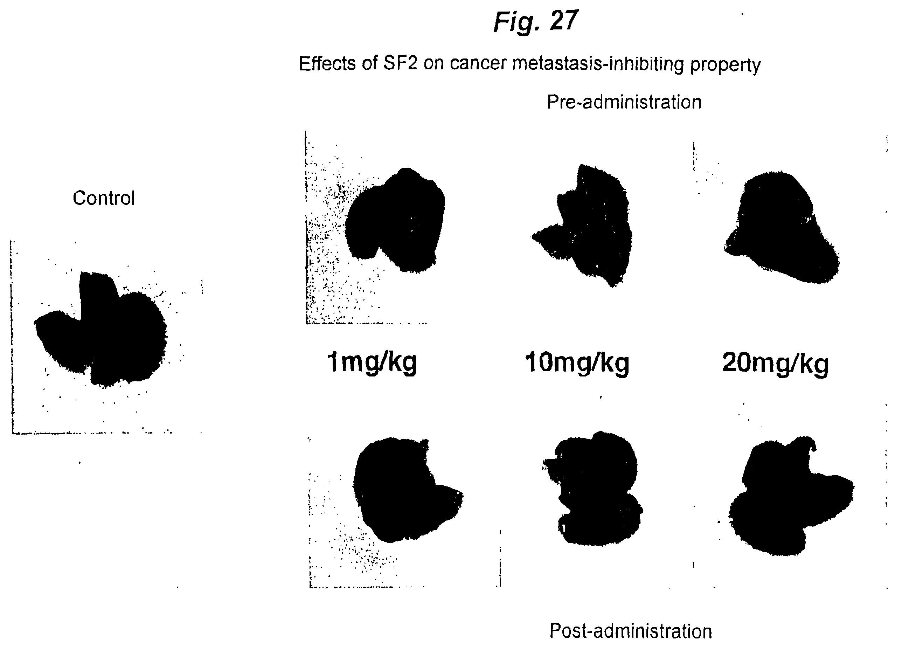

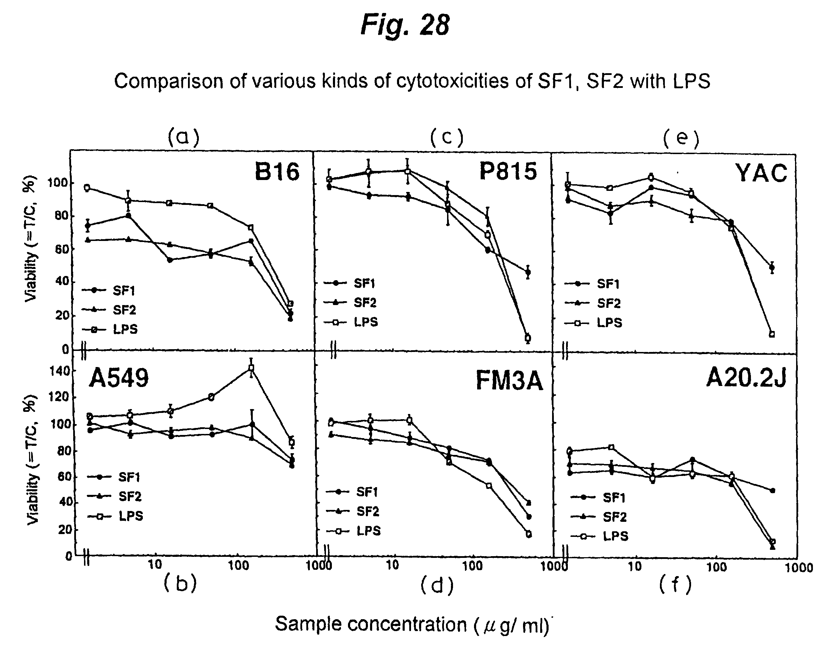

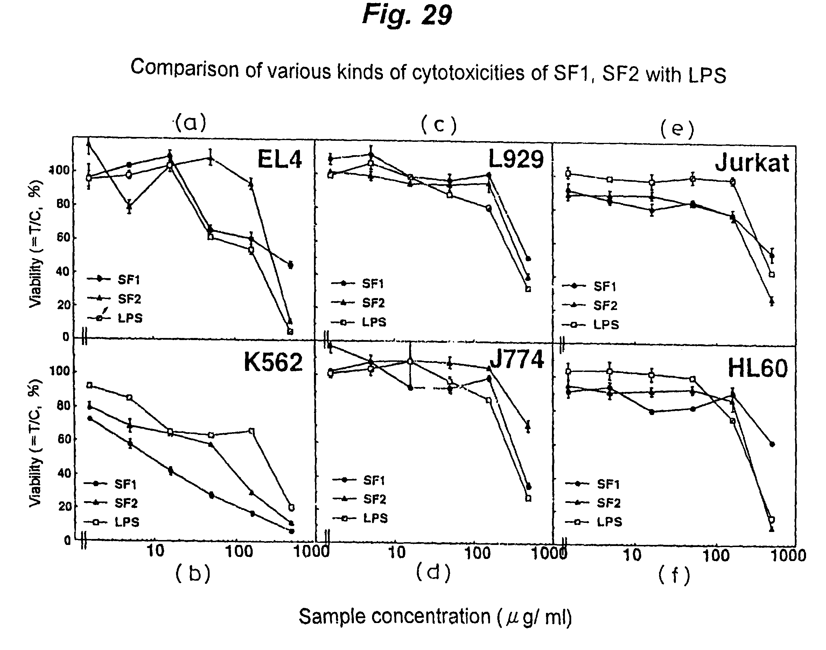

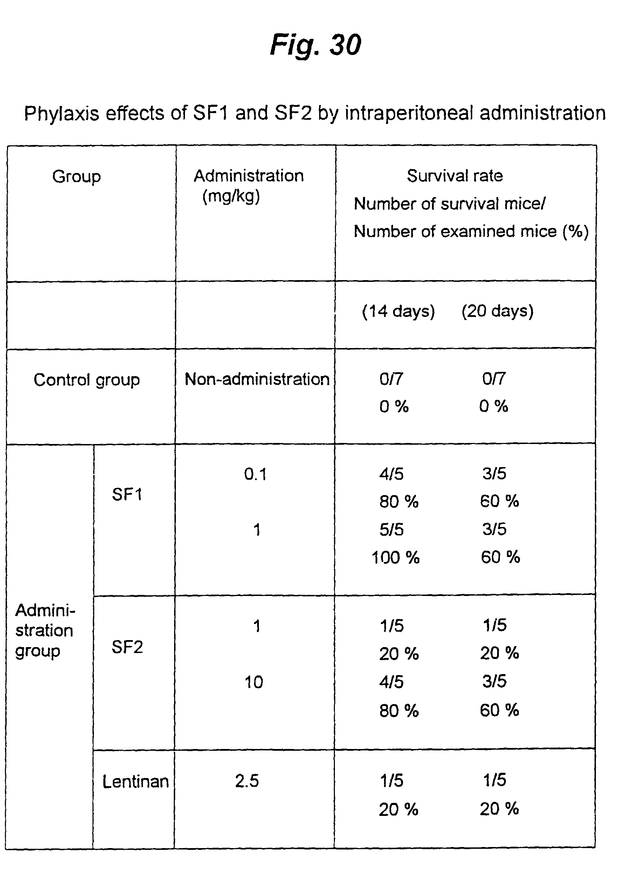

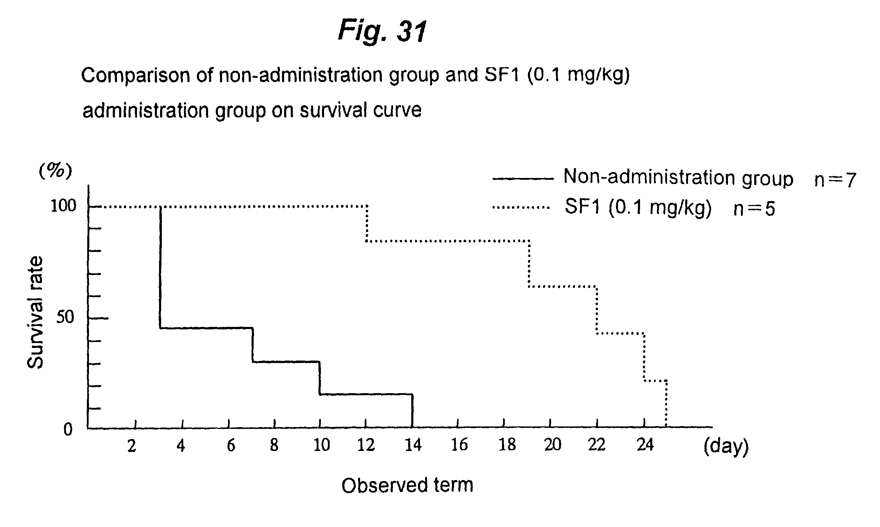

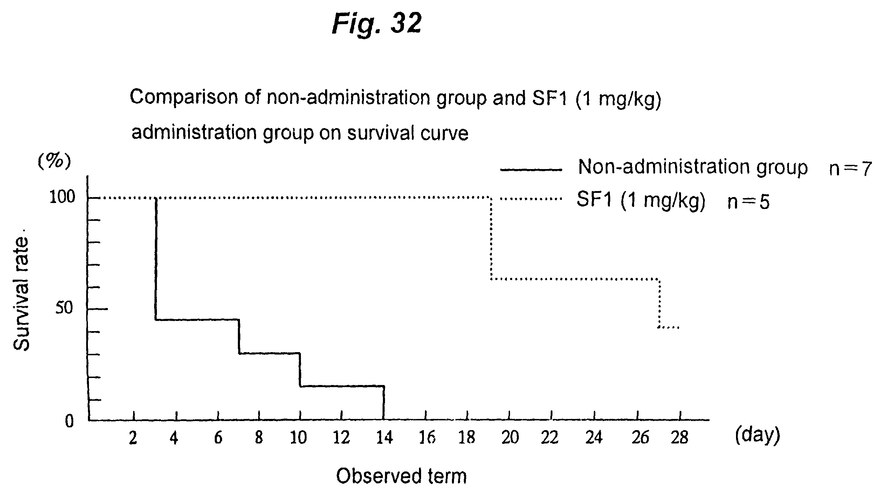

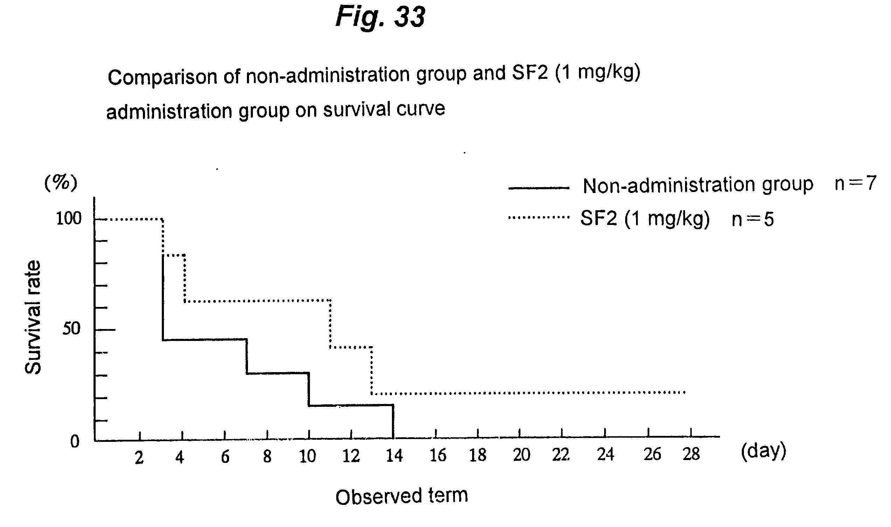

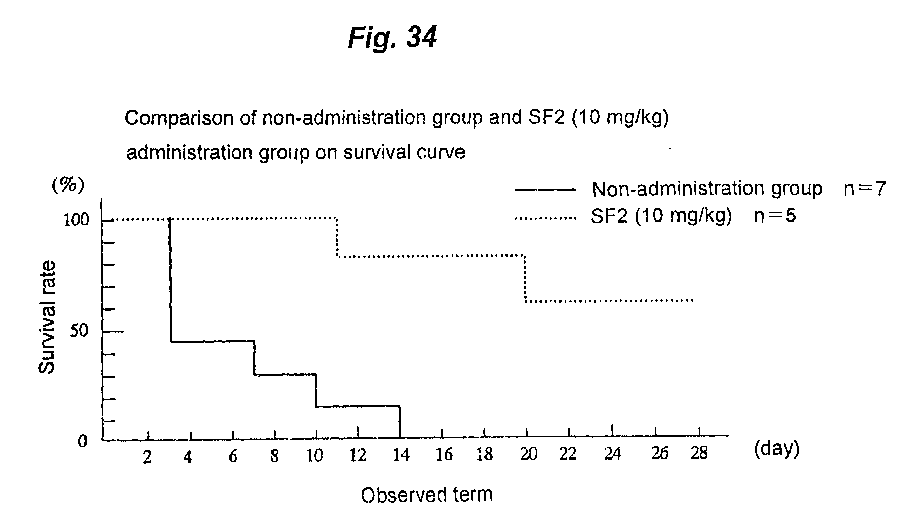

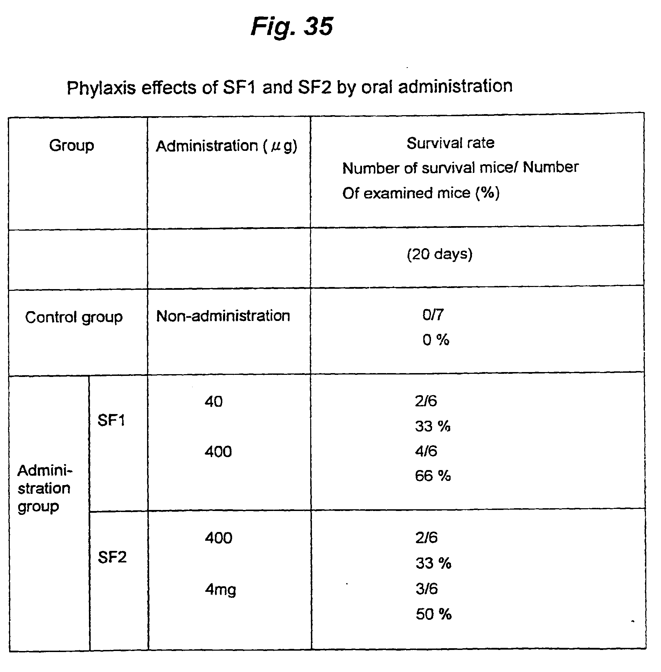

Fig. 1 is a flow chart showing an operation procedure of extracting a safflower with hot water which is explained in the embodiment of the present invention. Fig. 2 is a graph showing fractions state of a hot water extract of a safflower by gel filtration method. Fig. 3 is a table showing saccharide compositions of SF1 and SF2 in the hot water extracts of a petal of a safflower. Fig. 4 is a graph showing calibration curves to be used for determination of protein weight in SF1 and SF2. Fig. 5 is a flow chart showing preparation of splenocytes cells and procedure of inspection using their blastogenesis phenomenon and IgM production as indexes. Fig. 6 is a graph showing comparison of blastogenic activities of SF1 and SF2, and LPS. Fig. 7 is a graph showing comparison of IgM production activities of SF1 and SF2 in spleen B cells, with LPS. Fig. 8 is a flow chart showing preparation of PEC, and an inspection procedure of various kinds of physiologically active substances using the same. Fig. 9(a) is a flow chart showing procedure for inspecting production of IL-1, and Fig. 9(b) is a flow chart showing procedure for inspecting production of TNF-α. Fig. 10 is a graph showing comparison of production activities of IL-1 due to SF1 and SF2 in PEC, with LPS. Fig. 11 is a graph showing comparison of production activities of TNF-α due to SF1 and SF2 in PEC, with LPS. Fig. 12(a) is a flow chart showing procedure for inspecting production of IL-6, and Fig. 12(b) is a flow chart showing procedure for inspecting production of NO. Fig. 13 is a graph showing comparison of production activities of IL-6 due to SF1 and SF2 in PEC, with LPS. Fig. 14 is a graph showing comparison of production activities of NO due to SF1 and SF2 in PEC, with LPS. Fig. 15 is a table showing comparison of immunoactivity functions of SF1 and SF2. Fig. 16 is a table showing acute toxicity of SF1 and SF2 against mice. Fig. 17 is a flow chart showing procedure for inspecting effects of carcinostatic property of SF1 and SF2 by using ascites type of FM3A mouse breast cancer cells. Fig. 18 is a flow chart showing administration schedule of SF1 and SF2 when the effects of carcinostatic and cancer metastasis-inhibiting properties of SF1 and SF2 are to be inspected. Fig. 19 is a graph showing an effect of a carcinostatic action of SF1 in a mouse after FM3A was implanted. Fig. 20 is a graph showing an effect of a carcinostatic action of SF2 in a mouse after FM3A was implanted. Fig. 21(a) and (b) are graphs showing weight change of a mouse after implanting FM3A by post-administration of SF1 and SF2. Fig. 22 is a flow chart showing procedure for inspecting effects of cancer metastasis-inhibiting property of SF1 and SF2 by using melanoma B16 cells. Fig. 23(a) and (b) are graphs showing effects of cancer metastasis-inhibiting property of SF1 and SF2 at lung metastasis of B16 implanted mouse. Fig. 24 are photographs showing an effect of cancer metastasis-inhibiting property by pre-administration of SF1 and SF2 at lung metastasis of B16 implanted mouse. Fig. 25 are photographs showing an effect of cancer metastasis-inhibiting property by post-administration of SF1 and SF2 at lung metastasis of B16 implanted mouse. Fig. 26 are photographs comparing the effects of cancer metastasis-inhibiting property of SF1 at lung metastasis of B16 implanted mouse at pre-administration and post-administration. Fig. 27 are photographs comparing the effects of cancer metastasis-inhibiting property of SF2 at lung metastasis of B16 implanted mouse at pre-administration and post-administration. Fig. 28(a) is a graph showing cell toxicities of SF1 and SF2 in B16 cells in comparison with LPS, Fig. 28(b) in A549 cells, Fig. 28(c) in P815 cells, Fig. 28(d) in FM3A cells, Fig. 28(e) in YAC cells and Fig. 28(f) in A20.2J cells, respectively. Fig. 29(a) is a graph showing cell toxicities of SF1 and SF2 in EL4 cells in comparison with LPS, Fig. 29(b) in K562 cells, Fig. 29(c) in L929 cells, Fig. 29(d) in J774 cells, Fig. 29(e) in Jurkat cells and Fig. 29(f) in HL60 cells, respectively. Fig. 30 is a table showing phylaxis effects of SF1 and SF2 by intraperitoneal administration. Fig. 31 is a graph showing a comparison of survival curves between non-administered control group and SF1 (0.1 mg/kg) administered group. Fig. 32 is a graph showing a comparison of survival curves between non-administered control group and SF1 (1 mg/kg) administered group. Fig. 33 is a graph showing a comparison of survival curves between non-administered control group and SF2 (1 mg/kg) administered group. Fig. 34 is a graph showing a comparison of survival curves between non-administered control group and SF2 (10 mg/kg) administered group. Fig. 35 is a table showing phylaxis effects of SF1 and SF2 by oral administration.

### Best mode for carrying out the invention

To explain the present invention in more detail, it is explained in accordance with the attached drawings.

### (Hot water extraction of safflower)

First, isolation and purification of the fraction of a safflower extract are explained.

In the embodiment of the present invention, petals of a safflower made from China which is a plant member of a chrysanthemum family having a botanical name of Carthamus tinctorius L.

In the present embodiment, extraction of a safflower is carried out by the method of hot water extraction in which dried petals are extracted in hot water for one hour. After hot water extraction, the extract was cooled by allowing to stand, squeezed with a gauze, and applied to a centrifugal separator (J-25 rotor: JLA-10, 500, available from Beckmann Avanti Co.) at 10000xg for 20 minutes to remove insoluble residues.

To a supernatant obtained by removing insoluble residues was so added ethanol as to become the final concentration of 70%, thereafter the mixture was allowed to stand at -30°C one day and night to form precipitates. Such precipitates were separated by applying to the above-mentioned centrifugal separator at 10000xg for 10 minutes to separate.

The separated precipitates were suspended in 100% ethanol, and ethanol was evaporated by an evaporator (IWAKI REN-1 series, available from IWAKI Co.) to dry the precipitates.

The dried material was dissolved in distilled water and adjusted to pH 3 with hydrochloric acid and allowed to stand at 4°C overnight to effect acid precipitation. The acid precipitates were applied to the above-mentioned centrifugal separator at 10000xg for 10 minutes to separate from a supernatant.

The separated supernatant was adjusted to pH 7 with sodium hydroxide and ethanol was so added to become the final concentration of 70% to form precipitates. By using the above-mentioned evaporator, the precipitates were dried.

This dried material was dissolved in a 10 mM Tris-HCl (pH 7.4) to become 50 mg/ml, and insoluble substances were removed from the solution by using a filter paper under suction filtration to prepare an analytical sample for gel filtration mentioned below.

For gel filtration, XK50 (available from Pharmacia Biotech Co.) was used as a column, and Sepharose-CL2B (available from Pharmacia Biotech Co.) was used as a gel.

Using a 10 mM Tris-HCl buffer solution (pH 7.4) as an eluent, it is eluted with a flow rate of 2.5 ml/min. to fractionate 120 bottles with each 15 ml/Fraction. Fractionations of SF1 and SF2 were carried out using the mitogenic activities of the fractions as an index.

Using the above-mentioned column with a void volume of 40 ml, gel filtration was carried out with the above-mentioned manner, and the fraction numbers 40 to 50 are fractionated as SF1 and the fraction numbers 50 to 60 as SF2.

An outline of the operation procedure of extracting a safflower with hot water in the above-mentioned explanation is shown in Fig. 1 as a flow chart. Also, in Fig. 2, the state of fractionating the fraction numbers 40 to 50 which show remarkable absorption at the ultraviolet region of 280 nm as SF1, and the fraction numbers 50 to 60 as SF2. The dye specific for the safflower can be obtained in the range of the fraction numbers 79 to 105.

Incidentally, in Fig. 2, the fraction numbers are shown at the transverse axis and the absorbance at 280 nm and an amount of [³H]TdR taken in the cells are shown in the ordinates axis.

Moreover, the fractions fractionated by the above-mentioned manner are concentrated to about 10 times or so by evaporating the solvent by the above-mentioned evaporator, respectively, and ethanol is added thereto so as to become the final concentration of 70% to carry out formation of precipitation. Thereafter, the precipitates were separated by applying to a centrifugal separator (LC121, available from TOMY Co.) at 3000 rpm for 20 minutes to separate the precipitates. This precipitates were dissolved in a small amount of distilled water, and then freeze-dried by a freeze-drying machine (FRD-50M, available from IWAKI Co.) to prepare samples SF1 and SF2 for inspecting a carcinostatic action. Such freeze-dried SF1 and SF2 are white powders which are well dissolved in water, respectively.

Incidentally, when carcinostatic property, cancer metastasis-inhibiting action, immunoactivity, etc. are inspected by using SF1 and SF2 in the following, SF1 and SF2 are sometimes called to as samples for explanation purpose (which are the same as in graphs of the drawings).

In this connection, yields of SF1 and SF2 obtained by the hot water extraction in the above-mentioned explanation were about 5 mg in SF1 and about 50 mg in SF2 from 100 g of dried petals.

Next, determinations of saccharide compositions of SF1 and SF2 fractionated by the above-mentioned manner are explained.

To a weighed SF1 was added 2N trifluoroacetic acid to hydrolyze in a sealed tube. The mixture was cooled to normal temperature after hydrolysis and separated with ether. To the separated ether layer and the aqueous layer were blown with nitrogen to remove the solvents, and then, dried in a desiccator under reduced pressure.

Moreover, to the aqueous layer side were added a suitable amount of water and a NaBH₄ solution (prepared by dissolving 6 mg of NaBH₄ in 1N ammonia) and the mixture was reacted at 40 °C for 2 hours, then acetic acid was added to the mixture to remove excessive NaBH₄. Moreover, the solution was neutralized by adding Amberlite IR 12 OB (H⁺) which is an ion exchange resin and filtered. After filtration, the solvent was removed by evaporator. Furthermore, the precipitates were washed with methanol, and methanol and methyl boronate were removed and the precipitates were dried. After drying, pyridine and acetic anhydride were added to the precipitates to react at 60°C for 2 hours. Thereafter, water was added to the mixture to stop the reaction and dried. Thus, reduction of the aqueous layer side separated after hydrolysis of the fraction and acetylation of the same were successively carried out.

Also, when analyzing the saccharide composition mentioned below, 2-deoxy glucose was added to the above-mentioned fraction after hydrolysis and the mixture was used as an internal standard.

The dried product was dissolved in acetone, and 1 µl thereof is applied to GC and GC-MS analyses to determine the saccharide composition. In the same manner as mentioned above, saccharide composition of SF2 was also determined. The determined sacoharide compositions are shown in Fig. 3.

Incidentally, in the above-mentioned analysis, GC-14B type GC manufactured by Shimadzu Seisakusho and JMX-AX505HA manufactured by JOEL Co. were used as GC-MS. Also, ionization conditions of GC were carried out by ionizing with EI mode and 70 eV. An ionization room was set at 300°C. Also, apart from the saccharide composition of the macromolecular polysaccharide contained in the above-mentioned SF1 and SF2, elemental analyses of fractionated SF1 and SF2 were carried out.
SF1 had C: 32.7±0.1%, H: 5.81±0.1%, and N: 3.14±0.1%.
SF2 had C: 36.6±0.3%, H: 5.82±0.1%, and N: 2.03±0.1%.

For elemental analyses, automatic analyses were carried out by using MT-5 manufactured by Yanako Co.

Moreover, as for nitrogen found by the above-mentioned elemental analysis, presence of nitrogen was confirmed separately by the Bred-Ford determination method and Ninhydrin method. With regard to such a nitrogen, details thereof is not yet uncertain since the molecular weight and molecular structure of the substance itself of the present invention are not yet known, but it can be estimated at the present stage that a protein with a small molecular weight would be bound to the macromolecular polysaccharide having the saccharide composition shown in Fig. 3.

Particularly, it is estimated that the above-mentioned nitrogen would be derived from an amino acid either of aspartic acid, serine or threonine which constitutes the protein from the analysis thereafter.

As shown in Fig. 4, according to the above-mentioned Bred-Ford determination method, a calibration curve is prepared by using a bovine serum protein (BSA) as a standard substance, and amounts of the respective proteins were obtained from absorbances of SF1 and SF2 at 595 nm. An effective range of the calibration curve is made the range of 0<X≦2.5 µg/ml which range has good linearity from Fig. 4. In this determination method, experiment was carried out by using BIO-RAD protein assay kit (available from Bio Rad Co.).

A protein concentration from the calibration curve corresponding to the absorbance of 0.325 of SF1 at 50.926 µg/ml is 1.51 µg/ml and the protein content is about 3%.

A protein concentration from the calibration curve corresponding to the absorbance of 0.336 of SF2 at 160 µg/ml is 1.68 µg/ml and the protein content is about 1%.

Also, with regard to SF1 and SF2, even when they are treated with pronase, no change is observed in their activities so that it can be understood that the proteins found by the above-mentioned analysis at SF1 and SF2 have resistance to an enzyme. From the above facts, even after administration, SF1 and SF2 are not changed by an enzyme existing in a body to be administered and show the following pharmacological activities effectively.

### (Preparation of splenocytes and Immunoactivity test using its blastogenic tendency and IgM production as indexes)

First, with regard to SF1 and SF2, they were inspected by using blastogenic tendency and IgM production of extracted splenocytes of C57BL/6 mouse as indexes. The manner of inspection is as shown in the flow chart in Fig. 5, by effecting extraction of spleen from C57BL/6 mouse, and 1 x 10⁵ splenocytes were apportioned per well as a specimen and SF1 or SF2 was added thereto to examine blastogenic tendency and IgM production after addition thereof.

First, preparation of the splenocytes is carried out by extracting spleen from a slaughtered C57BL/6 mouse by decapitation, squeezing the spleen in a 3 ml of MEM (modified Eagle medium) and suspended well by a Pasteur's pipette. Thereafter, after removing cell mass by a metal mesh, cells were collected by a low speed centrifugation (200xg, 3 minutes). After removing supernatant, the precipitated cell mass was loosened, 2 ml of a 10 mM Tris-HCl buffer solution (pH 7.4) containing 0.83% of NH₄Cl was added thereto and the mixture was allowed to stand for 2 minutes to effect hemolysis. After hemolysis, the mixture was washed three times with the above-mentioned MEM, then suspended in PRMI 1640 + 10% FCS medium to make 2 x 10⁵ cells/ml. The thus prepared splenocytes were applied for inspecting blastogenic tendency and IgM production hereinbelow.

As for blastogenic tendency, each 50 µl of the SF1 and SF2 samples and the above-mentioned cells suspension was applied to a 96-wells plate, cultured for 48 hours, and observation by a microscope with the lapse of 48 hours was carried out. In addition, measurement of a mitogenic activity was also carried out.

For measurement of the mitogenic activity, 158 MBq of thymidine in which ³H is introduced into a hydrogen of the methyl group was diluted to 0.925 MBq with purified water, and it is added before 4 hours of completion of culture so as to become 9.25 kBq/well. Thereafter, by using a cell harvester (available from Semi.12 Skatron Co.), cells were collected on the glass filter. After drying, cells were placed in a vial, 1 ml of toluene series scintillator was added thereto, and an amount of ³H-Thymidine taken into the cells was measured by a liquid scintillation counter (S6500, available from Beckmann Co.).

Also, LPS was used in the same manner as in SF1 and SF2 as a control. The results are shown in the graph of Fig. 6. It can be understood that there are effects of blastogenesis in the order of SF1>SF2>LPS when the used concentrations of SF1 and SF2 become around 100 µg/ml.

Also, with regard to IgM production, as shown in Fig. 7, it was inspected by using the produced immunoglobulin M and examining the absorbance at the visible region of 415 nm by using an ELISA measurement method (enzyme immunoassay method).

In the inspection manner as in the above-mentioned blastogenesis, each 50 µl of the SF1 and SF2 samples and the above-mentioned cell suspension was applied to a 96-well plate, cultured for 72 hours, and IgM amounts in the culture supernatants were inspected by the ELISA method. For inspection, anti-mouse IgM rabbit antibody is used as a primary antibody and a peroxidase labeled anti-mouse IgM rabbit antibody is used as a second antibody.

As the result, as shown in the graph of Fig. 7, it can be understood that at around 100 µg/ml at which blastogenic activity is strengthened as shown in Fig. 6, SF1 and SF2 show substantially the same IgM production activity, and thereafter activities become greater than that of LPS.

According to the above results, it can be understood that SF1 and SF2 which are safflower extracted substances have the same or superior effects to that of LPS in the blastogenic activity and IgM production activity, respectively.

### (Preparation of PEC and immunoactivity test by inspection of production of various kinds of physiologically active substances)

According to the above-mentioned experiments, it was confirmed that SF1 and SF2 both have effects in blastogenesis of cells and IgM production, and immunoactivities by production of the other physiologically active substances were also inspected.

First, as shown in the flow chart of Fig. 8, 3.8% thioglycolate medium was injected in intraperitoneal cavity of C3H/HeN mouse with 2.5 ml/head, and PEC which is exudate cells in the mouse intraperitoneal cavity recovered from intraperitoneal cavity after 4 days is used as a TG induced macropharge. The number of cells of this PEC was adjusted to 2 x 10⁵ cells/ml in the PEMI 1640 + 10% FCS medium, and further apportioned to a 24-well plate with 500 µl, and 500 µl of a sample was added thereto and the cells were cultured in an incubator (BNA-111, available from ESOPEC Co.) for 24 hours. The supernatant was metastasisred into an Eppendorf tube, the mixture was subjected to centrifugation at 5000 rpm for one minute and the supernatant was again recovered and inspections of productions of the following physiologically active substances were carried out.

As production of various kinds of physiologically active substances, inspections were carried out with regard to production abilities of IL-1 (Interleukin 1), TNF-α (Tumor necrosis factor), IL-6 (Interleukin 6), and NO which is a free radical chemical species.

First, with regard to production of IL-1, as shown in the flow chart of Fig. 9(a), thymocytes of C3H/HeJ mouse were extracted, to the cells was added PHA so as to become a final concentration of 2 µg/ml, and thereafter, 5 x 10⁵ cells were apportioned per one well. This was added to the supernatant of cultured PEC prepared in the manner as mentioned above, and production of IL-1 was inspected by examining an amount of the labeled [³H]TdR with the lapse of 48 hours.

Preparation of the thymocytes is carried out by extracting thymus from a slaughtered C3H/HeJ mouse by decapitation, squeezing the thymus in a 3 ml of MEM (modified Eagle medium) and suspended well by a Pasteur's pipette. Thereafter, after removing cell mass by a metal mesh, cells were collected by a low speed centrifugation (200xg, 3 minutes). Moreover, cells were washed twice with MEM, then, suspended in PRMI 1640 + 10% FCS medium to adjust the cell solution so as to become 1 x 10⁷ cells/ml. PHA was added to the cell solution so as to become 2 µg/ml, and each 50 µl of a sample and the cell solution was applied to 96-well plate and cultured for 48 hours.

Moreover, as in the measurement of the mitogenic activity, 158 MBq of thymidine to which ³H is introduced into a hydrogen of the methyl group was diluted to 0.925 MBq with purified water, and it is added before 4 hours of completion of culture so as to become 9.25 kBq/well. Thereafter, by using a cell harvester (available from Semi.12 Skatron Co.), cells were collected on the glass filter. After drying, cells were placed in a vial, 1 ml of toluene series scintillator was added thereto, and an amount of ³H-Thymidine taken into the cells was measured by a liquid scintillation counter (S6500, available from Beckmann Co.).

Incidentally, the same experiment was carried out by using recombinant mouse IL-1 and a calibration curve was prepared. Based on the calibration curve, IL-1 in the culture supernatant of macropharge was determined.

The results of IL-1 thus determined are shown in the graph of Fig. 10.

It can be understood that production of IL-1 (in Fig. 10, the produced amount is shown by ng/ml) by SF1 becomes larger than LPS which is used as a control at the concentration of exceeding 10 µg/ml or so. On the other hand, it can be understood that production of IL-1 by SF2 is smaller than LPS.

Next, with regard to production of TNF-α, as shown in the flow chart of Fig. 9(b), 0.75 x 10⁵ cells/well of L929 cells were added to the supernatant of PEC culture cells so as to the final concentration of actinomycin D of 2 µg/ml, and after 18 hours lapsed, cells were stained by Crystal violet and absorbance thereof was measured at the visible region of 595 nm. The number of unit of TNF-α per 1 ml was calculated from the absorbance, and the results are shown in the graph of Fig. 11.

Preparation of L929 cells was carried out as follows. First, L929 cells were adjusted so as to 1.5 x 10⁶ cells/ml by PRMI 1640 + 10% FCS medium, and actinomycin D was added so as to become 2 µg/ml. Each 50 µl of a sample and the prepared L929 cells suspension was added to the 96-well plate, and cultured in the above-mentioned incubator for 18 hours. The supernatant was removed, each 100 µl of PBS (phosphate buffer solution) was added for washing, 50 µl of 0.2% Crystal violet (methanol solution) was added and after allowed to stand for 15 minutes to dissolve it, and then, the mixture was washed with water. After drying the plate, 100 µl of methanol was added thereto to dissolve the stain solution, thereafter measurement of an absorbance was carried out at 595 nm by a microplate reader (MODEL 450, BIO-RAD Co.).

Also, calculation of a produced amount of TNF-α was carried out and calculated by using that in which L929 cells are cultured by PRMI 1640 + 10% FCS medium as a positive control (100%), that cultured by 1% SDS (sodium dodecyl sulfate) as a negative control (0%), and a sample concentration which provides 50% of cell death as 1 unit.

The produced amount of TNF-α calculated by the above-mentioned manner is shown in the graph of Fig. 11. Production of TNF-α is, as can be seen from Fig. 11, both of SF1 and SF2 show remarkable producing abilities than LPS as a control at a concentration greater than 100 µg/ml, but SF2 was remarkably low in producing ability than SF1.

Also, with regard to production of IL-6, as shown in the flow chart of Fig. 12(a), 5 x 10³ cells/well of MH60 cells are added to PEC culture supernatant in the same manner as mentioned above, and after 48 hours lapsed, inspection of the producing ability was carried out by using a MTT reagent.

Preparation of MH60 cells which are dependent cells of IL-6 is carried out firstly by washing three times with PRMI 1640 + 10% FCS medium, and adjusted so as to become 1 x 10⁵ cells/ml in PRMI 1640 + 10% FCS medium. Each 50 µl of a sample and the cell suspension were applied to a 96-well plate and cultured for 48 hours. Before completion of culture, a MTT reagent (5 mg/ml) was added with 10 µl/well, and after culturing for 4 hours. 100 µl of a 20% SDS aqueous solution was added. Thereafter, the mixture was allowed to stand in an incubator overnight to effect culture. After completion of culture, absorbance at 595 nm was measured by a microplate reader.

Incidentally, for effecting determination, a calibration curve was prepared by effecting the same experiments as mentioned above using recombinant IL-6, and IL-6 in a culture supernatant of macropharge was determined from the calibration curve.

The results show that, as shown in the graph of Fig. 13, it can be understood that SF1 has higher producing ability than that of LPS which is a control over the whole range of the concentration. On the other hand, it can be understood that SF2 has the highest producing ability near to 100 µg/ml but below the concentration range than the above, its producing ability thereof is lower than that of LPS. However, in either of the cases, it can be understood that SF1 and SF2 have producing ability of IL-6, particularly SF1 is larger than SF2.

Moreover, with regard to production of NO (nitrogen monoxide) which is a free radical chemical spices, as shown in the flow chart of Fig. 12(b), after adding 10% Griess Romijn reagent (Griess Romijn Nitrite Reagent) to PEC culture supernatant in the 96-well plate, and allowed to stand at room temperature for 10 minutes, and color formation was measured by measuring absorbance at the visible region of 545 nm. The produced amount of NO was determined by measuring the NO in the culture supernatant of macropharge as an amount of NO₂ based on a calibration curve which was prepared by carrying out the similar experiment as mentioned above by using sodium nitrite (NaNO₂).

The results show that, as shown in the graph of Fig. 14, it can be understood that up to 100 µg/ml, NO producing ability of SF1 is larger than that of SF2. However, it can be understood that in the range, NO producing ability of SF1 is larger than that of SF2 up to 100 µg/ml. However, in the range, it can be understood that the NO producing ability is lower than the that of LPS as a control.

The results in which inspection of production about various kinds of physiologically active substance were carried out are shown briefly in Fig. 15.

### (Acute toxicity test to mouse)

For effecting inspection of carcinostatic action of SF1 and SF2 by using BDF1 mouse and C3H/HeN mouse, acute toxicity tests to the above-mentioned mice were previously carried out to determine the value of LD₅₀. The results are shown in the following Fig. 16.

### (Inspection of carcinostatic action)

With respect to the carcinostatic action, as shown in the flow chart of Fig. 17, 1 x 10⁵ cells were implanted to C3H/HeN mouse by injecting mouse breast cancer ascites type FM3A cells into intraperitoneal cavity and the action was inspected by a survival ratio and weight increase of mice.

For effecting inspection, in accordance with an administration schedule shown in the flow chart of Fig. 17, tests were carried out by dividing into a pre-administration group in which SF1 or SF2 is administered before implantation and a post-administration group in which they are administered after implantation, and the results are compared to each other.

In the pre-administration group, SF1 or SF2 is intraperitoneally injected once a day over 5 days to C3H/HeN mouse, and FM3A cells were implanted in the order shown in the flow chart shown in the above-mentioned Fig. 17, and the survival days were examined.

In the post-administration group, FM3A cells were implanted to C3H/HeN mouse in the order shown in the flow chart shown in Fig. 18, and one day later, SF1 or SF2 was intraperitoneally injected once a day over 5 days and the survival days were examined.

The results of survival days in the pre-administration group and the post-administration group are shown in the graphs of Figs. 19 and 20.

In the graph of Fig. 19, with regard to SF1, it can be understood that the post-administration group shows longer survival days than the pre-administration group. Also, particularly in the case of post-administration, the survival rate is the highest in the case of an administration dose of 0.1 mg/kg, and there is a tendency that the survival rate is lowered as the administration dose is increased as 1 mg/kg and 10 mg/kg.

Also, in the graph of Fig. 20, the results regarding SF2 are shown. As in the graph of Fig. 19, the post-administration shows better effects than that of the pre-administration, and it can be understood that the survival rate is lowered in the order of the administration dose of 1 mg/kg, 10 mg/kg and 20 mg/kg.

Also, in the case of the post-administration, as shown the results in the graph of Fig. 21, change in the weight increase was examined. In the graph shown in Fig. 21(a), the results of examining the tendency of weight increase by SF1 day by day are shown. As shown in the graph, abrupt increase and abrupt decrease in weight are initially observed, but this is only a passing weight increase due to increase in cancer cells, and when the effects of SF1 start to appear, then abrupt decrease in weight due to abrupt decrease in cancer cells is observed. Thereafter, normal weight increase is observed, and survival of mouse for 70 days was confirmed. With regard to SF2, the similar tendency is observed as shown in the graph of Fig. 21(b).

From the results as mentioned above, it can be understood that SF1 and SF2 derived from safflower extracts have carcinostatic actions.

### (Inspection of cancer metastasis-inhibiting action)

With regard to cancer metastasis-inhibiting action, melanoma B16 cells are implanted to BDF1 mouse and inspection about metastasize to lung was carried out. The melanoma B16 cells are, as shown in the flow chart of Fig. 22, implanted by intravenous injection with a ratio of 4 x 10⁵ cells/head from a tail portion of mouse to prepare a specimen. After lapsing 16 days from implantation, mice were slaughtered to effect extraction of lung, and cancer metastasis-inhibiting properties of SF1 and SF2 were inspected by the number of colonies of the melanoma B16 cells metastasized to lung.

First, specimens were divided into a pre-administration group and a post-administration group in the same manner as in the inspection manner of the above-mentioned carcinostatic action, and the effects were compared to each other.

In the pre-administration group, SF1 or SF2 is intraperitoneally injected once a day over 5 days to the above-mentioned mouse, and then, melanoma B16 cells were implanted in the order as mentioned above.

On the other hand, in the post-administration group. after implanting melanoma B16 cells to the above-mentioned mouse, and one day later, SF1 or SF2 was intraperitoneally injected once a day over 5 days. Such an administration schedule is described in the flow chart shown in Fig. 18.

For effecting inspection, in the both groups of the pre-administration group arid the post-administration group, mice were slaughtered 16 days elapsed after the implantation to extract lung, and the number of colonies of the melanoma B16 cells was counted.

The results are shown in Fig. 23. From Fig. 23(a), with regard to SF1, it can be understood that the pre-administration is small in the number of colonies in the extracted lung than the case of the post-administration. That is, with regard to SF1, as far as cancer metastasize, at least lung metastasis is concerned, it can be understood that the pre-administration shows higher effect than the post-administration. Also, as shown in Fig. 23(b), in SF2, it can be understood that the pre-administration is effective than the post-administration.

Such effects of administration doses for cancer metastasis-inhibiting properties are shown in the photographs of Fig. 24 and Fig. 25 with the lapse of time. In the photographs of Fig. 24, the states of cancer metastasis-inhibiting properties are shown in the cases of pre-administrations of SF1 and SF2. With regard to SF1, administration doses are divided into three degrees of 0.1 mg/kg, 1 mg/kg and 10 mg/kg, and with regard to SF2, administration doses are divided into three degrees of 1 mg/kg, 10 mg/kg and 20 mg/kg, and experiments are carried out.

In the photographs of Fig. 25, in the cases of the post-administrations of SF1 and SF2, under the same conditions as shown in the photographs of Fig. 24, cancer metastasis-inhibiting properties were examined.

As can be clearly seen from the both photographs, it can be clearly seen that the pre-administration (shown in the photographs of Fig. 24) shows less number of colonies (in the photographs, they are photographed as black dots) of melanoma B16 cells metastasized to lung than the post-administration (shown in the photographs of Fig. 25).

Moreover, in the photographs of Fig. 26 and the photographs of Fig. 27, the results obtained with regard to SF1 and SF2 are shown with enlarged photographs to easily understand the above results. In the both photographs, as in the same as the above results, in either of the cases of the pre-administration or the post-administration, they show effects as compared with the non-administered control, but it can be understood that the pre-administration shows particularly remarkable effects than the post-administration. That is, colonies (black dots) of melanoma B16 are far little in the pre-administration than in the post-administration, particularly in SF1, it can be understood that when pre-administration is carried out with an administration dose of 10 mg/kg, colony is disappeared.

In the photographs shown in Fig. 27, as inspected in the photographs of the above-mentioned Fig. 26, also in SF2, it can be understood that the pre-administration shows potent cancer metastasis-inhibiting property than in the case of the post-administration.

### (Inspection of cytotoxicity)

Also, with regard to cytotoxicities of SF1 and SF2 against various kinds of cells, they are inspected by using LPS as a control. Cells to be inspected are 12 cells in total including B16, P815, YAC, A549, FM3A, A20.2J, EL4, L929, Jurkat, K562, J774 and HL60 cells and their cytotoxicities were inspected.

For effecting inspection, respective cells are adjusted to become 1 x 10⁵ cells in PRMI 1640 + 10% FCS medium, each 50 µl of a sample and a cell suspension is applied to 96-well plate and they are cultured for 48 hours. Before 4 hours of completion of culture, MTT reagent (5 mg/ml) was added with 10 µl/well. Then, after culturing for 4 hours, 100 µl of a 20% SDS aqueous solution was added and allowed to stand in an incubator overnight. After completion of culture, measurement of absorbance at 595 nm was carried out by a microplate reader.

Those culturing with PRMI 1640 + 10% FCS medium alone are made a control (100%), and a viability of cells is calculated.

For effecting inspection, with regard to SF1, SF2 and LPS which is a control, it is carried out by measuring viability (T/C, %) of cells by changing an administration dose (µg/ml) variously. Such states are shown by both graphs of Fig. 28 and Fig. 29.

### (Inspection of phylaxis property)

In the experiments of phylaxis property, Candida albicans of clinically separated strain TIMM 1768 was intravenously injected to 5-weeks old female ICR mouse which is uninfected with a specific pathogen, and phylaxis properties of SF1 and SF2 against the above were examined.

In the experiments, the day before inoculating the above-mentioned Candida albicans to a mouse, 200 mg/kg of cyclophosphamide which is an immunosuppressive agent was previously intraperitoneally injected.

In addition, from the day when the above-mentioned cyclophosphamide is intraperitoneally injected, a predetermined amount (see Fig. 30) of SF1, SF2 or commercially available Lentinan (Comparison) is each intraperitoneally injected separately into the mouse to which the above-mentioned cyclophosphamide was administered for continuously for 4 days to form administration groups. Moreover, as a control group, a non-administration group (control group) in which cyclophosphamide alone is administered and administration of SF1, etc. is never carried out is formed.

Then, after continuous administration of SF1, SF2 or commercially available Lentinan (Comparison) for 4 days, the above-mentioned Candida albicans was inoculated at the 5th day to the mice of the administration groups and the non-administration group, states of the mice with regard to the administration groups to which SF1, SF2 or Lentinan (Comparison) is each administered and the non-administration group to which SF1, SF2 or Lentinan is never administered were observed for 28 days to examine survival rates of mice.

In Fig. 30, survival rates at the 14th day and 20th day of the above-mentioned respective administration groups and non-administration group are shown.

From Fig. 30, whereas the survival rate of the non-administration control group is 20% at the 14th day, it can be understood that high survival rates are shown in the SF1 administration group of 80 to 100% at the 14th day and 60% at the 20 day, and in the SF2 administration group of 20 to 80% at the 14th day and 20 to 60% at the 20th day. In the administration group of Lentinan, the survival rate was 20% (at the 14th day, at the 20th day).

It can be understood that administrations of 0.1 mg/kg and 1 mg/kg are particularly effective in SF1, and 10 mg/kg is particularly effective in SF2. Moreover, in both of SF1 administration group and SF2 administration group, when an administration dose is increased 10-fold, a survival rate is increased and the state wherein the survival rate depends on the administration dose is observed whereby effectivenesses of SF1 or SF2 is confirmed.

Moreover, the activity in such a phylaxis property was not admitted in Lentinan which is a carcinostatic polysaccharide so that such phylaxis activities are considered to be characteristic features of SF1 and SF2.

Also, in Figs. 31 and 32, comparison of survival curves of the administration group of SF1 and the control group (non-administration group) is shown. In Figs. 33 and 34, comparison of survival curves of the administration group of SF2 and the control group (non-administration group) is shown. In either cases of the survival curves of SF1 or SF2, significant difference can be admitted when judged with a level of significance of 1% with the significant test of Wilcoxson. In the significant test of Cox-Mantel, significant difference could be also admitted when judged with a level of significance of 1%, and it was confirmed that effects of phylaxis properties by SF1 and SF2 are effective.

Next, separately from administration by the above-mentioned intraperitoneal injection, effects of SF1 and SF2 by oral administrations were also inspected.

In this experiments, mice and clinically separated strains are the same as in the case of administration by the above-mentioned intraperitoneal injection.

In the experiments, before the inoculation date of the above-mentioned Candida albicans to mice, cyclophosphamide which is an immunosuppressive agent was previously intraperitoneally injected in an amount of 200 mg/kg.

In addition, each predetermined amount (see Fig. 35) of SF1 or SF2 was suspended in 0.2 ml of distilled water and they were each orally administered everyday to separate mice continuously for 4 days from the intraperitoneal injection date of the above-mentioned cyclophosphamide to form an administration group. As a control, intraperitoneal injection of cyclophosphamide alone was carried out to form a non-administration group in which oral administration of SF1, etc. had been never carried out.

Thereafter, at the 5th day after continuous oral administration of SF1 or SF2 for 4 days, the above-mentioned Candida albicans was inoculated to the mice of the administration group and the mice of the non-administration group. Then, survival rates of the mice of the administration group to which SF1 or SF2 had been each orally administered and that of the non-administration group to which SF1 or SF2 had been never orally administered were examined for 28 days.

In Fig. 35, survival rates at the 20th day of the above-mentioned administration group and non-administration group were shown. From Fig. 35, it can be understood that whereas the survival rate of the control group of non-administration at the 20th day is 0%, the SF1 administered group shows a high survival rate of 33 to 66% and the SF2 administration group of 33 to 50%.

Particularly in SF1, it can be understood that oral administration of 400 µg shows clear phylaxis activity and shows a high survival rate. Also, in the cases of administrations of 40 µg of SF1, 400 µg and 4 mg of SF2, they show high survival rates and their effectivenesses can be inferred whereas they are not equal to the case of administration of 400 µg of SF1.

From Fig. 35, in both of the SF1 administration group and the SF2 administration group, it can be understood the tendencies when the administration doses are increased 10-folds, survival rates are also remarkably increased. Thus, the survival rates depend on the administration doses so that it can be said that the oral administrations of SF1 or SF2 shown by the present experiments are effective.

Incidentally, with regard to such phylaxis activities in the above-mentioned SF1 and SF2, details of their mechanisms are not yet known but the above-mentioned experimental results show that SF1 and SF2 inhibit growth of Candida in a living body and it is considered to suggest that there is a possibility that SF1 and SF2 break the vicious circle of the infection and lowering in immune observed in an AD (atopic dermatitis) patient.

Moreover, inspection experiments of phylaxis effects of SF1 and SF2 against various kinds of bacteria and molds are now still continuing, but it is expected that phylaxis properties against MRSA, staphylococcus, Pseudomonas aeruginosa, etc. would be effective since they show effective effects against inveterate Candida albicans infection as mentioned above.

### (Preparation)

In the following, preparation of the pharmacologically active substances, SF1 and SF2, obtained as the above-mentioned extracts of safflower.

For making preparation of such SF1 and SF2, each can be considered to form a preparation of an internal agent or an injection agent. An administration dose when constituting as an injection agent or an internal agent is as follows.

Incidentally, the administration dose shown below shows an average administration dose with regard to an adult person, and thus, it is necessary to suitably modify the administration dose depending on the subject to be administered such as an age, body weight, symptom, whether conceived or not, etc. of the subject to be administered.

In SF1, when it is used as an internal agent, it can be used within the range of 50 mg to 5 g/person. As clearly seen from the various experiments such as the above-mentioned carcinostatic action, etc., the effect of SF1 is confirmed with 10 mg/kg so that when adult weight is made 60 kg and considering a safety ratio, the minimum dose per day is made 1/10 amount of the effective dose for animals, and the maximum of a diary dose is made an internal dose of the usual Chinese medicine and set.

Also, when SF2 is used as an internal agent, 10-fold amount of SF1 can be administered as a rule, but for example, it may be set within the range of 200 mg to 10 g/person.

When SF1 is used as an injection agent, it may be 1/10 of the internal dose and may be administered within the range of 5 to 500 mg/person. In the case of SF2, as in SF1, it may be set within the range of 20 mg to 1 g/person which is 1/10 administration dose of the above-mentioned internal agent of SF2.

When they are administered as injection agents in the above-mentioned administration range, they can be used in the form of subcutaneous injection agents, intramuscular injection agents or intravenous injection agents.

### I) Internal agent

As the internal agents which contain SF1 or SF2 as an effective ingredient, they can be provided as an internal solid agent or an internal liquid agent. When they are provided as the internal solid agent, the agent form can be selected various forms such as tablets, capsules, granules, powder, pills, etc., and it is particularly preferred to form an enteric preparation.

By forming the internal agent as an enteric preparation, the effective ingredient is more rapidly entered into blood by intestinal absorption, and as a result, progress of production of IL-1, IL-6, TNF-α, etc. can be rapidly urged. According to the facts, immunoactivity action, phylaxis action, carcinostatic action, and cancer metastasis-inhibiting action, can be effectively developed.

For example, when it is provided as a tablet, an excipient described in a pharmacopoeia such as saccharides (e.g., glucose), starches (e.g., corn), etc., a binder described in a pharmacopoeia such as gum Arabic, polyvinyl alcohol (PVA), etc., a disintegrating agent such as starches, etc., a glossy agent, described in a pharmacopoeia such as magnesium stearate, etC., is(are) added depending on necessity to SF1 or SF2 and mixed, and a tablet having a predetermined shape may be formed by tabletting using a tablet preparing method such as the direct powder pressing method, the dry granule pressing method, the wet granule pressing method, etc.

In particular, when an enteric coating tablet is to be prepared, a film coating such as cellulose acetate phthalate (CAP), hydroxypropylmethyl cellulose phthalate (HPMCP), etc. as an enteric film base agent may be carried out.

### i) Main prescription example of internal solid agent

### (1): Prescription example of enteric coating tablet using SF1 (composition in one tablet)

| | |
|---|---|
| SF1 | 0.05 g |
| D-mannitol (excipient) | 0.1 g |
| PVA (binder) 0.1 g | |
| Magnesium stearate(glossy agent) | 0.3 g |
| Cellulose acetate phthalate (coating agent) | 0.1 g |

The above mixture are made an enteric tablet by the direct powder pressing method.

### (2): Prescription example in which SF1 is made powder (composition in one package)

| | |
|---|---|
| SF1 | 0.05 g |
| D-mannitol (excipient) | 0.5 g |
| White sugar | 0.8 g |

The above mixture are made separated packages depending on the administration dose.

### (3): Prescription example in which SF1 is made a granular agent (composition in one package)

| | |
|---|---|
| SF1 | 0.05 g |
| D-mannitol (excipient) | 2 g |

Incidentally, the granular agent with the above-mentioned composition may be previously coated with an enteric coating agent described in a pharmacopoeia such as cellulose acetate phthalate to make it enteric.

### (4): Prescription example in which SF1 is made a hard capsule agent

| | |
|---|---|
| SF1 | 0.05 g |
| D-mannitol (excipient) | 0.8 g |

The granular agent with the above-mentioned composition is filled in a hard capsule using gelatin described in a pharmacopoeia as a base material.

### (5): Prescription example in which SF1 is made a soft capsule agent

| | |
|---|---|
| SF1 | 0.05 g |
| Water | 1.5 g |

The medical solution with the above-mentioned composition is encapsulated into a soft capsule made of a gelatin base material to which glycerin or sorbitol is added.

### (6): Prescription example of enteric coating tablet using SF2 (composition in one tablet)

| | |
|---|---|
| SF2 | 0.5 g |
| D-mannitol (excipient) | 0.8 g |
| PVA (binder) | 0.5 g |
| Magnesium stearate(glossy agent) | 0.5 g |
| Cellulose acetate phthalate (coating agent) | 0.8 g |

The above mixture are made an enteric tablet by the direct powder pressing method.

### (7): Prescription example in which SF2 is made powder (composition in one package)

| | |
|---|---|
| SF2 | 0.5 g |
| D-mannitol (excipient) | 3 g |
| White sugar | 6 g |

The above mixture are made separated packages depending on the administration dose.

### (8): Prescription example in which SF2 is made a granular agent (composition in one package)

| | |
|---|---|
| SF2 | 0.5 g |
| D-mannitol (excipient) | 2 g |

Incidentally, the granular agent with the above-mentioned composition may be previously coated with an enteric coating agent described in a pharmacopoeia such as cellulose acetate phthalate to make it enteric.

### (9): Prescription example in which SF2 is made a hard capsule agent

| | |
|---|---|
| SF2 | 0.5 g |
| D-mannitol (excipient) | 0.5 g |

The granular agent with the above-mentioned composition is filled in a bard capsule using gelatin described in a pharmacopoeia as a base material.

### (10): Prescription example in which SF2 is made a soft capsule agent

| | |
|---|---|
| SF2 | 0.5 g |
| Water | 0.5 g |

The medical solution with the above-mentioned composition is encapsulated into a soft capsule made of a gelatin base material to which glycerin or sorbitol is added.

Incidentally, other than the above-mentioned internal solid agent, it may be provided as a liquid state, the so-called internal liquid agent, which gives good bioavailability easily obtained. As such an internal liquid agent, it may be provided as a liquid agent, a suspending agent, an emulsion, an elixir agent, a syrup agent, etc. described in a pharmacopoeia.

### ii) Medical effects of internal agent

The internal agents of the above-mentioned SF1 and SF2 show immunoactivities as clearly seen from the above-mentioned various kinds of experiments, particularly as for development of the immunoactivity, progresses of production of IL-1 and IL-6 are confirmed. Thus, even in the body in which a power of immune is failing, the present internal agent can be used as an immune activating agent which is expected to have the effects such as production promoting effects of IL-1 and IL-6, improvement and advancement of the power of immune, etc., IL-1 production promoting effect and IL-6 production promoting effect.

Also, SF1 and SF2 show preventing properties against infection other than the above-mentioned immunoactivities from the above-mentioned animal experiments. Thus, even in the case where the preventing property against infectious bacteria is failed due to lowering in the power of immune, etc., the present internal agent can be used as a phylaxis agent which previously prevents infection due to bacteria, etc.

SF1 and SF2 show production promoting effect of TNF-α, and effects of complete destruction of cancer occurred, inhibition of generating cancer, inhibition of growth of cancer, inhibition of metastasis of cancer to the other portion, etc., so that the present internal agent can be used as a TNF-α production promoting agent, carcinostatic agent, cancer metastasis-inhibiting agent in the body.

### II) Injection agent

As an injection agent, SF1 and SF2 both show good water-solubility so that, for example, each of the predetermined amount is dissolved in water for injection described in a pharmacopoeia and can be provided in the form in which it is sterilized and sealed in an ampoule. For providing it as such an aqueous injection agent, it may be so adjusted that an administration dose at one time can be carried out within the range of 1 to 10 ml. Such an injection agent can be provided as subcutaneous injection agents, intramuscular injection agents, intravenous injection agents, etc.

Incidentally, SF1 or SF2 is dissolved in the solution in which a harmless non-aqueous solvent such as ethanol or propylene glycol described in a pharmacopoeia is mixed with water and provided as an injection agent.

Moreover, by considering the case where the providing injection agent is used by dividing some portions, a preservative may be mixed. If necessary, a stabilizer may be mixed to obtain chemical and physical stabilities of the aqueous injection agent of SF1 or SF2. Also, to decrease pain at injection, an analgesic agent may be mixed.

Also, except for providing as an ampoule injection agent, powder of SF1 or SF2 and water as a solvent are each provided by separate ampoules (or vial), and may be used by dissolving the powder when it is to be used. In this case, an excipient may be added to SF1 or SF2 doses of which are small to increase the amount whereby to make their handling easy.

### i) Main prescription example of injection agent

### (1): Prescription example of aqueous injection agent containing SF1 as effective ingredient

| | |
|---|---|
| SF1 | 0.005 g |
| Water for injection | 2 g |

The above mixture is provided as 2 ml of an ampoule.

### (2): Prescription example of an injection agent which dissolved at the time of using containing SF1 as effective ingredient

| | |
|---|---|
| SF1 | 0.005 g |
| Sorbitol (bulking agent) | 2 g |

The above powder preparation is encapsulated in an ampoule (or vial).

In combination with the above-mentioned powder preparation, 2 ml of water for injection described in a pharmacopoeia is encapsulated in a separate ampoule (or vial) as a dissolving agent and provided.

### (3): Prescription example of aqueous injection agent containing SF2 as effective ingredient

| | |
|---|---|
| SF2 | 0.05 g |
| Water for injection | 2 g |

The above mixture is provided as 2 ml of an ampoule.

### (4): Prescription example of an injection agent which dissolved at the time of using containing SF2 as effective ingredient

| | |
|---|---|
| SF2 | 0.05 g |
| Sorbitol (bulking agent) | 2 g |

The above powder preparation is encapsulated in an ampoule (or vial).
In combination with the above-mentioned powder preparation, 3 ml of water for injection described in a pharmacopoeia is encapsulated in a separate ampoule (or vial) as a dissolving agent and provided.

### ii) Medical effects of injection agent

The injection agents of the above-mentioned SF1 and SF2 show immunoactivities as clearly seen from the above-mentioned various kinds of experiments, particularly as for development of the immunoactivity, progresses of production of IL-1 and IL-6 are confirmed. Thus, even in the body in which a power of immune is failing, the present injection agent can be used as an immune activating agent which is expected to have the effects such as production promoting effects of IL-1 and IL-6, improvement and advancement of the power of immune, etc., IL-1 production promoting effect and IL-6 production promoting effect.

Also, SF1 and SF2 show preventing properties against infection other than the above-mentioned immunoactivities from the above-mentioned animal experiments. Thus, even in the case where the preventing property against infectious bacteria is failed due to lowering in the power of immune, etc., the present injection agent can be used as a phylaxis agent which previously prevents infection due to bacteria, etc.

SF1 and SF2 show production promoting effect of TNF-α, and effects of complete destruction of cancer occurred, inhibition of generating cancer, inhibition of growth of cancer, inhibition of metastasis of cancer to the other portion, etc., so that the present injection agent can be used as a TNF-α production promoting agent, carcinostatic agent, cancer metastasis-inhibiting agent in the body.

In the above-mentioned explanation, explanation was made human beings as an object to be administered, but other than human beings, it can be applied to, for example, mammals including domestic animals such as cattle, horses, etc., birds including domestic fowls such as chickens, domestic ducks, etc., various kinds of vertebral animals such as fishes as objects to be administered. In such cases, preparation forms suitable to the objects to be administered are optionally selected and preparations are made. For example, it can be provided as a powder agent or a granular agents which can be internally administered by mixing with feed or fodder, etc., or an injection agent such as an intraperitoneal injection agent.

### (Used example for food)

SF1 and SF2 which are pharmacologically active substance obtained as extracts of the above-mentioned safflower can be used for foods. Particularly, SF1 and SF2 do not lost their activities against pronase treatment so that they are stable against various enzymatic reactions at digestion and addition to foods is effective.

However, when they are used for foods, a used amount of a crude drug for YAKUZEN (a meal combined with a herbal medicine and normal food materials for improving health or preventing and curing a disease), etc. can be employed as a standard, but different from medical products, considering the fact that they are usually taken so that used examples in which used amounts are suppressed to a little amount as compared with the above-mentioned preparations are shown below.

### i) Used example for corn flake

### (1): Used example of SF1

To 870 g of corn grits, 110 g of liquid sugar and 20 g of table salt is added 0.3 g of SF1 and they are mixed. Thereafter, according to the conventional method, through the respective steps of steaming, molding and drying, corn flake in which 0.01 g of SF1 is formulated per 35 g of one meal is prepared.

### (2): Used example of SF2

To 870 g of corn grits, 110 g of liquid sugar and 20 g of table salt is added 1.5 g of SF2 and they are mixed, and then, according to the conventional method, through the respective steps of steaming, molding and drying, corn flake in which 0.05 g of SF1 is formulated per 35 g of one meal is prepared.

### ii) Used example for instant noodle

### (1): Used example of SF1

To 890 g of flour, 100 g of starch and 10 g of table salt is added 0.12 g of SF1 and they are mixed, and according to the conventional method, the above-mentioned mixture is subjected to the respective steps of rolling, cutting down, steaming, cutting for each one meal, and hot air drying to prepare a non-fried instant noodle to which 0.01 g of SF1 is formulated per 80 g of one meal.

### (2): Used example of SF2

To 890 g of flour, 100 g of starch and 10 g of table salt is added 0.6 g of SF2 and they are mixed. Thereafter, 350 ml of water is added to the mixture and they are mixed, and then, according to the conventional method, the above-mentioned mixture is subjected to the respective steps of rolling, cutting down, steaming, cutting for each one meal, and hot air drying to prepare a non-fried instant noodle to which 0.05 g of SF2 is formulated per 80 g of one meal.

In the above explanation, used examples for foods with regard to corn flake and non-fried instant noodle are shown, but the foods are not limited only to them and they can be used for foods other than the above.

As mentioned above, the invention accomplished by the present inventor is concretely explained based on the embodiments of the invention, but the present invention is not limited to the above-mentioned forms and it would be needless to say that in the range which does not deviate the summary thereof can be variously changed.

### Utilizability in industry

As mentioned above, the pharmacologically active substances of the present invention show immunoactivities, IL-1 production promoting, IL-6 production promoting, TNF-α production promoting, carcinostatic action, cancer metastasis-inhibiting action, and phylaxis action, etc., and suitable for using as a medicine. Moreover, they can be applied to cosmetics, food additives using such a pharmacologically active substance, etc., in the field of cosmetics, the field of food hygiene, etc.

## Claims

1. A pharmacologically active substance which comprises a macromolecular polysaccharide comprising rhamnose, arabinose, xylose, mannose, glucose and galactose.

2. The pharmacologically active substance according to Claim 1, wherein said pharmacologically active substance is an extract of a safflower.

3. The pharmacologically active substance according to Claim 1, wherein 2.9 of rhamnose is contained in a weight ratio to 1.0 of mannose.

4. An immunoactive agent which comprises a macromolecular polysaccharide comprising rhamnose, arabinose, xylose, mannose, glucose and galactose.

5. A carcinostatic agent which comprises a macromolecular polysaccharide comprising rhamnose, arabinose, xylose, mannose, glucose and galactose.

6. A cancer metastasis-inhibiting agent which comprises a safflower extract as an effective ingredient.

7. A phylaxis agent which comprises a macromolecular polysaccharide comprising rhamnose, arabinose, xylose, mannose, glucose and galactose.

8. An IL-1 production promoting agent which comprises a macromolecular polysaccharide comprising rhamnose, arabinose, xylose, mannose, glucose and galactose.

9. An IL-6 production promoting agent which comprises a macromolecular polysaccharide comprising rhamnose, arabinose, xylose, mannose, glucose and galactose.

10. A TNF-α production promoting agent which comprises a macromolecular polysaccharide comprising rhamnose, arabinose, xylose, mannose, glucose and galactose.

11. A food which comprises a pharmacologically active substance defined in any one of Claims 1 to 3 being added thereto.
